# EUROPEAN PATENT SPECIFICATION

(11) **EP 3 129 385 B1**
(45) Date of publication and mention of the grant of the patent: **06.06.2018**
(21) Application number: 15718442.5
(22) Date of filing: 10.04.2015
(51) Int. Cl.: C07D 493/08, C07D 493/18, A61K 31/365, A61P 29/00, A61P 31/12, A61P 37/00

(54) **NEOSORAPHENS AND THEIR USE IN THE TREATMENT OR PREVENTION OF A VIRAL INFECTION OR OF A TH17-ASSOCIATED INFLAMMATORY AND/OR AUTOIMMUNE DISEASE**
NEOSORAPHENE UND IHRE VERWENDUNG ZUR BEHANDLUNG ODER VORBEUGUNG EINER VIRALEN INFEKTION ODER EINER MIT TH17 ASSOZIIERTEN ENTZÜNDUNGS- UND/ODER AUTOIMMUNERKRANKUNG
NÉOSORAPHÈNES ET LEUR UTILISATION POUR LE TRAITEMENT ET À LA PRÉVENTION D'UNE INFECTION VIRALE OU D'UNE MALADIE INFLAMMATOIRE ET AUTO-IMMUNE ASSOCIÉES À TH17

(30) Priority: 11.04.2014 EP 14001331
(43) Date of publication of application: 15.02.2017
(73) Proprietor: Helmholtz-Zentrum für Infektionsforschung GmbH, 38124 Braunschweig (DE); Twincore Zentrum für Experimentelle und Klinische Infektionsforschung GmbH, 30625 Hannover (DE)
(72) Inventor: MUELLER, Rolf, 66440 Blieskastel (DE); HUEHN, Jochen, 38100 Braunschweig (DE); STEINMETZ, Heinrich, 31139 Hildesheim (DE); KECK, Matthias, 10437 Berlin (DE); HARMROLFS, Kirsten, 66123 Saarbrücken (DE); SPARWASSER, Tim, 30625 Hannover (DE); LOCHNER, Matthias, 30163 Hannover (DE); NANDAN, Amrita, 30625 Hannover (DE); KOUTSOUDAKIS, George, 08014 Barcelona (ES); MEYERHANS, Andreas, 08013 Barcelona (ES); DIEZ, Juana, 08013 Barcelona (ES); MARTINEZ, Javier, 08005 Barcelona (ES); BRÖNSTRUP, Mark, 38102 Braunschweig (DE); KRUG, Daniel, 66111 Saarbrücken (DE)
(74) Representative: Forstmeyer, Dietmar
(86) International application number: PCT/EP2015/000757
(87) International publication number: WO 2015/154883

(56) References cited:
- EP-A2- 2 581 081
- WO-A1-03/011867
- KIRA J. WEISSMAN ET AL: "Myxobacterial secondary metabolites: bioactivities and modes-of-action", NATURAL PRODUCT REPORTS, vol. 27, no. 9, 1 January 2010 (2010-01-01), page 1276, XP055128338, ISSN: 0265-0568, DOI: 10.1039/c001260m

## Description

### Field of the Invention

The present invention relates to compounds according to general formula (I); to compositions, including a pharmaceutical formulation and a combination preparation comprising one or more of the compound(s); to a process for their preparation; them for uses, including the use in the treatment or prevention of a viral infection or a Th17-associated inflammatory and/or autoimmune disease.

### Background of the Invention

### T cells

T cells (or thymocytes) are a class of lymphocytes, which play a key role in the defense against foreign pathogens and autoimmunity in mammals. T-cells originate from hematopoietic stem cells formed in the bone marrow and are released into blood and lymph. Different types of T cells having diverse roles in the function of the immune system are recognized; broadly speaking, T cells can be divided into the following types: T helper (Th) cells, cytotoxic T cells (CTLs), memory T cells (TMs), regulatory T cells (Treg cells), and natural killer T cells (NKT cells).

T-helper cells (Th cells) are a subset of αβT-cells that usually express the CD4 co-receptor and have a major role in controlling and regulating the immune system by providing 'help' to other white blood cells. Accordingly, CD4 Th cells play a central role in initiating and maintaining immune responses. They have a crucial influence on the nature of an ongoing immune response, which can be either regulatory or inflammatory. Upon stimulation of the T-cell receptor, naive CD4+ T helper cells have the potential to differentiate into functionally different T helper cell lineages. Classically, Th1 cells express Interferon-y and play an important role in regulation of cellular immunity, while Th2 cells express Interleukin 4, 5 and 13 and are involved in regulation of humoral immunity and the response against extracellular parasites. Recently, a third T helper cell lineage, Th17 cells, which produce the highly inflammatory cytokines IL17A and IL17F and other pro-inflammatory cytokines, was identified. The pro-inflammatory cytokines and chemokines expressed by Th17 cells target both immune and non-immune cell types and Thl7 cells have been shown to play an important role in a variety of inflammatory and autoimmune diseases, e.g. psoriasis, rheumatoid arthritis, multiple sclerosis, inflammatory bowel disease and asthma (reviewed, e.g. in Korn, T. et al., Annu Rev Immunol 27, 485-517 (2009); Tesmer, L. A. et al., Immunol Rev 223, 87-113 (2008); Crome, S. et al., Clin Exp Immunol 159(2), 109-19 (2009); and Annunziato, F. et al., Trends Immunol, PMID: 22682163 (2012)).

Regulatory T cells, or 'Tregs' are fundamental in controlling various immune responses in that Tregs can rapidly suppress the activity of other immune cells. In particular, Tregs are crucial for the induction and maintenance of self-tolerance and for the prevention of autoimmunity (see, e.g. Sakaguchi, S., Annu Rev Immunol 22, 531-62 (2004)). For instance, Treg defects have been discovered in patients with multiple sclerosis (MS), type I diabetes (T1D), psoriasis, myasthenia gravis (MG) and other autoimmune diseases (Baecher-Allan, C. & Hafler, D. A., Immunol Rev 212, 203-16 (2006)). Similar links may also exist for atopy and allergic diseases (Robinson, D. S., Larche, M. & Durham, S. R., J Clin Invest 114, 1389-97 (2004)). For all these diseases reports exist pointing to a reduced immune suppression of the patient's Treg cells. This has led to an increasing interest in the possibility of using Tregs in immunotherapy to treat or prevent chronic infections, autoimmune diseases, allergies and transplantation-related complications, such as graft rejection or graft-versus-host disease (GvHD) (for a review, see Roncarolo, M. G. & Battaglia, M., Nat Rev Immunol 7, 585-98 (2007)). The characteristic marker of Tregs is Foxp3.

### Acetyl-coenzyme A carboxylase (ACC)

Acetyl-coenzyme A carboxylases (ACCs) (EC 6.4.1.2) catalyze the first rate limiting step in fatty acid synthesis - the ATP dependent carboxylation of acetyl-CoA to produce malonyl-CoA. As a result, ACCs are important regulators of fat metabolism. Eukaryotic ACCs are large, multi-domain enzymes that exist in humans and other animals in two isoforms, ACC1 and ACC2. ACC1 is expressed in lipogenic tissues (e.g. liver, adipose, lactating mammary gland and others). In these tissues the malonyl-CoA produced by ACC is used in the biosynthesis of long-chain fatty acids by the fatty acid synthase complex (FAS). The long-chain fatty acids can then be incorporated into triacylglycerides and phospholipids. In contrast, ACC2 is expressed mostly in the heart and skeletal muscle, and in these tissues malonyl-CoA functions mainly as a potent inhibitor of fatty acid oxidation. Due to their important role in fat metabolism, ACCs have been suggested as targets for drugs (for a review, see, e.g. Tong L., Cell Mol Life Sci 62, 1784-1803 (2005)).

Potent and selective inhibitors of acetyl-coenzyme A carboxylase, including ACC1 and ACC2, (herein also referred to as ACC inhibitors) have been developed and reported as agents for the treatment of a variety of diseases, including obesity, type 2 diabetes mellitus, Alzheimer's disease, and cancer. Generally speaking, ACC inhibitors are known in the art and have been reported in the literature. For instance, ACC inhibitors and uses thereof are described in US 7956049, US2006/765530, US 2007/671150, US 2009/466639A, US 2009/466639, US 7652023, US 2005/739344, US 2006/560388, US 7553836, US 2006/765530, US 2007/671150, US 7371759, US 2003/505893, US 2004/946055, US 2012/0108619, US 2010/408127, US 2011/531744, US 2011/0230461, US 2010/315522, US 2011/444401, US 2011/0003759, US 2011/0003767, US 2010/0009982, US 2007/948213, US 2008/141228, US 2010/881234, US 2010/0280067, US 2009/174152, US 2009/247620, US 2010/771019, US 2010/0113473, US 2008/109746, US 2009/582325, US 2010/0035892, US 2007/902594, US 2009/543792, US 2006/765530, US 2007/671150, US 2009/466639, EP 2189458, US 2008/109746, WO 2012/056372, US 2010/408127, US 2011/0531744, WO 2012/090219, WO 2010/127212, WO 2010/127208, WO 2008/103382, EP 0 359 706; Tong and Harwood, J Cell Biochem 99, 1476-1488 (2006); and Corbett J. W., Expert Opin Ther Patents 19(7), 943-956 (2009), and exemplary ACC inhibitors include one or more of any of the following compounds: 1*S*,2*S*,3E,5R,6*S*,11*S*,14*S*,15R,16R,17*S*,18*S*)-15,17-dihydroxy-5,6,16-trimethoxy-2,14,18-trimethyl-11-phenyl-12,19-dioxabicyclo[13.3.1]nonadec-3-en-13-one (Soraphen A; disclosed e.g. in EP 2581081 A2, WO 03/011867 A1 or Weissman K. J. et al., Nat. Prod. Reports 27(9), 1276-1295 (2010)) ; 5-(tetradecyloxy)-2-furan-carboxylic acid (TOFA); CP-640186 [(3R)-1-[1-(anthracene-9-carbonyl)piperidin-4-yl]piperidin-3-yl]-morpholin-4-ylmethanone]; CP-610432 (S-1'-(anthracene-9-carbonyl)-N,N-diethyl-1,4'-bipiperidine-3-carboxamide); CP-610431 (R-1'-(anthracene-9-carbonyl)-N,N-diethyl-1,4'-bipiperidine-3-carboxamide); CP-497485 (1'-(anthracene-9-carbonyl)-N,N-diethyl-1,4'-bipiperidine-3-carboxamide); phenylmethyl 5-(1-{[(2-{[N-(2,4-dihydroxy-3,3-dimethylbutanyl)-5-(6-aminooctahydro-9H-purin-9-yl)-4-(hydroxyl-2-[(phosphonooxy) tetrahydrofuran-2-yl]methyl dihydrogen diphosphate-β-alanyl]amino}ehyl)thio]acetyl}-2-oxohexahydro-1H-thieno[3,4-d]imidazol-4-yl)pentanoate; 3,3-dimethylhexanoate, monoglyceride (AC-0417-9); MEDICA 16 (β,β,β',β'-tetramethylhexadecanoic acid); ESP-55016 (8-hydroxy-2,2,14,14-tetra-methylpentadecanediotic acid); S2E ((+)-p-[1-p-tert-butylphenyl)-2-oxo-4-pyrrolidinyl]methoxybenzoic acid); 1'-N-Chloroacetamidobiotin, benzyl ester (CABI); and analogues and derivatives thereof.

Some of these ACC inhibitors have also been reported to have an antiviral effect on certain viruses. For instance, Gaunt et al. (J Gen Virol, 94, 1310-1317 (2013); DOI 10.1099/ vir.0.050146-0) found that TOFA reduces the infectivity of progeny rotavirus (RV) 31-fold and viral RNA production 6-fold. The effect of TOFA on RV infectivity and RNA replication was dose-dependent, and infectivity was reduced by administering TOFA up to 4 h post-infection. For human cytomegalovirus (HCMV) a similar effect of TOFA has been demonstrated (Spencer et al., J Virol 85(12), 5814-5824 (2011); doi:10.1128/JVI.02630-10).

### Rheumatoid arthritis (RA)

RA has long been classified as a Th1-mediated disease, but is now also thought to be a primarily Th17-driven disease. Initial evidence for a pathogenic role of IL-17 in RA came from reports that IL-17 was increased in the sera and synovial fluids of RA patients. As with psoriasis, there is also increased IL-22 and IL-23 in the synovium of RA patients. Notably, the increase in IL-17 and IL-23 appears to be specific for RA, because a similar increase was not found in patients with osteoarthritis (Crome et al., loc. cit. (2009)).

### Multiple sclerosis (MS)

There is also a strong association between IL-17 and MS (Tesmer *et al*., loc. cit. (2008)). Studies in mice have shown that Th17 cells play a critical role in both the induction and progression of EAE, the murine model of MS. In humans with MS it is not clear whether IL-17 mediates its potentially pathogenic effects directly or via recruitment of other immune cells. Human blood-brain barrier (BBB) endothelial cells from MS patients express the receptors for IL-17 and IL-22, and exposure of these cells to IL-17, but not IL-22, results in low expression of tight junction proteins and increased transmigration of CD4+ T cells. Moreover, IL-17 elevates expression of matrix metalloproteinase expression, leading to BBB dysfunction and neuronal apoptosis. Thus, a major pathological mechanism in MS could be IL-17-mediated destruction of the BBB allowing easier access of myelin-specific T cells to this usually immune privileged site (from Crome et al., loc. cit. (2009)).

### Psoriasis

Psoriasis is a chronic inflammatory skin disease that results in epidermal hyper- plasia, dermal angiogenesis and infiltration of monocytes, DCs and T cells. Both Th1 and Th17 cells are implicated in the pathogenesis, as there are elevated levels of both Th1- and Thl7-associated cytokines in serum and lesional skin. The most powerful evidence for the pathogenic role of Th17 cells in psoriasis comes from a clinical study which found that antibody-mediated blockade of the shared IL-23/IL-12 p40 subunit is an effective treatment: 81% of patients had at least a 75% improvement compared to only 2% of patients who received placebo. Targeting TNF-a is also an effective treatment for this disease, and improved clinical outcomes in this context are also associated with down-regulation of many Th17-associated cytokines including IL-17, IL-22, IL-6, IL1b and IL-23, in addition to CCL20 and anti-microbial peptides (from Crome *et al.,* loc. cit. (2009)).

### Inflammatory bowel disease (IBD), Ulcerative colitis, Crohn's disease

There is also evidence for the pathological role of Th17 cells in inflammatory bowel disease. In this disease there are genetic, correlative and therapeutic data which all support the hypothesis that Th17 cells drive intestinal inflammation. A large study of Europeans of different ethnicities found that a single nucleotide polymorphism in a non-coding region of the IL-23R was significantly associated with both Crohn's disease and ulcerative colitis. CD4+ T cells from peripheral blood and tissues of Crohn's disease patients express high levels of IL-17, with up to 40% co-expressing IFN-g and clustering in the lamina propria. Additionally, high IL-17 is found in sera and colonic biopsies of Crohn's disease patients (from Crome et al., loc. cit. (2009)).

### Asthma

In those patients with asthma in which inflammation is nonatopic, non-IgE-dependent, and noneosinophilic, airway neutrophilia is correlated with asthma severity, suggesting a major role for neutrophils, at least in this subset of patients with asthma. Neutrophilic inflammation has also been described in sudden-onset fatal asthma and neutrophil numbers are highly elevated in status asthmaticus, thus suggesting a possible role for these cells in severe and fatal asthma. As the role of IL-17 in neutrophil recruitment to the airways is well known, in the last years several studies tried to find an association between Th17 lymphocytes and asthma. Animal models of asthma suggest that Th17 cells promote neutrophilic inflammation, and, in concert with Th2 cells, are important in the development of airway hyper-responsiveness. Moreover, allergic sensitization through the airway promotes Th17 responses, and IL-17F-deficient mice have an impaired neutrophilic response to allergen. More importantly, in humans increased expression of IL-17A and IL-17F has been shown in bronchial submucosa of moderate to severe asthma, and the evaluation of induced sputum in patients with asthma revealed that neutrophilic inflammation was frequently present, particularly in the severe forms of disease. Furthermore, it has been reported that increased AHR in response to methacholine in patients with asthma positively correlates with IL-17A levels in the sputum. Finally, a polymorphism in IL-17F that results in a loss-of-function mutation is inversely related to asthma risk. (from Cosmi et al., Allergy 66(8), 989-98 (2011)).

### Type I diabetes (not type II diabetes)

Reports have shown the upregulation of Th17 immunity (IL-17, RORC, and IL-22) and Tregs related marker FOXP3 in *ex vivo* derived peripheral blood memory CD4⁺ cells and as response to anti-CD3 plus anti-CD28 stimulation in peripheral blood mononuclear cells in children with newly diagnosed type 1 diabetes (Honkanen et al., J Immunol 185(3), 1959-67 (2010)). Moreover, it was found that IL-17 contributes to apoptosis in human pancreatic islets and mouse insulinoma cells *in vitro,* as measured by up-regulated gene expression of stress related genes iNOS, SOD2, COX-2 and down regulated gene expression of anti-apoptotic BCL-2. The role of IL-17 as a cytokine mediating beta-cell death was further supported by a recent report, which also demonstrated IL-17 pathway activation as response to islet cell derived antigens (Arif et al., Diabetes 60(8), 2112-9 (2011). Marhawa et al. showed that children with new-onset type 1 diabetes (T1D) have an increased proportion of CD45RA-CD25intFOXP3low cells that are not suppressive and secrete significantly more IL-17 than other FOXP3⁺ subsets ( Marwaha et al., J Immunol 185(7), 3814-8 (2010)), which supported the plasticity of Th17 cells in human type 1 diabetes. Moreover, these diabetic subjects had a higher proportion of both CD4⁺ and CD8⁺ T cells that secrete IL-17. Several studies suggest that IL-17 cells in human type 1 diabetes secrete combinations of cytokines, such as IL-17, IFN-gamma and IL-9 (Beriou et al., J Immunol 185(1), 46-54 (2010)).

Currently treatment of Th17-associated inflammatory and autoimmune diseases, e.g. rheumatoid arthritis, psoriasis, systemic sclerosis, systemic lupus erythematosus, asthma, atopic dermatitis, contact hypersensitivity, multiple sclerosis, autoimmune myocarditis, type I diabetes, autoimmune thyroiditis, inflammatory bowel disease, Crohn's disease, ulcerative colitis, transplant rejection, is often not effective, as there is no therapy that stops or prevents the development of Th17-associated inflammatory and autoimmune diseases directly. Similar considerations apply to the treatment of many viral infections, where treatment is often limited to the treatment of the symptoms such as fever, pain, runny nose or diarrhea, since viruses have no metabolism of their own, and accordingly are difficult to control without simultaneously damaging the body's cells. Against some viral infections, e.g. influenza (flu), herpes, and HIV, antivirals that directly target the viral pathogen are known.

As a result, a need remains to provide easily applicable methods and compounds or compositions that may be used to effectively treat Th17-associated inflammatory and autoimmune diseases or viral infections.

It is, therefore, an aim of the present invention to provide novel compounds according to general formula (I); a pharmaceutical formulation comprising at least one of said compounds; a combination preparation comprising at least one of said compounds; a process for their preparation as well as them for use in the treatment or prevention of Th17-associated inflammatory and autoimmune diseases or viral infections. Preferably, such treatment is more effective and not as burdensome as current treatments and improves the lives of the patients.

### Summary and Description of the Invention

The present invention was made in view of the prior art and the needs described above, and, therefore, the object of the present invention is to provide a novel alternative compound for use in treating and/or preventing a viral infection, a Th17-associated inflammatory disease, and/or a Th17-associated autoimmune disease.

Other objects of the present invention are to provide a pharmaceutical composition comprising at least one compound of the invention, a combination preparation comprising at least one compound of the invention and at least one further active pharmaceutical ingredient, and a compound of the invention for use as a medicament. Further objects of the present invention are to provide a compound, a pharmaceutical composition or a combination preparation according to the invention for use in the treatment or prevention of a viral infection, a Th17-associated inflammatory disease, and/or a Th17-associated autoimmune disease, and a method for the preparation of a compound according to the invention.

These objects are solved by the subject matter of the attached claims.

These and other aspects of the present invention will become apparent upon reference to the following detailed description and definitions.

The present invention relates to a compound of the general formula (I): 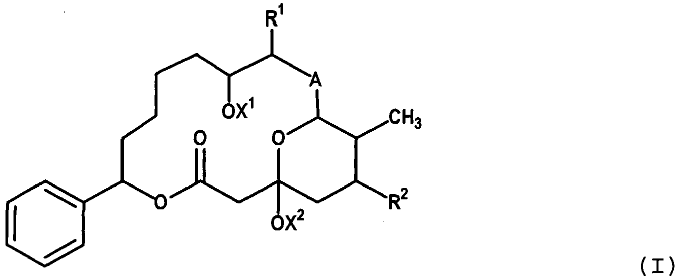 or a pharmacologically acceptable salt thereof, wherein A represents a group of the formula: 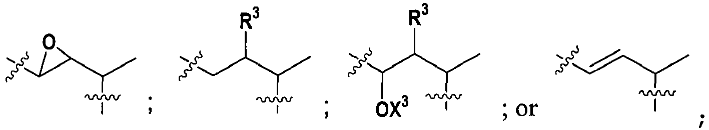
R¹, R² and R³, in each case, independently represents a hydrogen atom, a halogen atom, CN, CF₃, NO₂, NH₂, -NHC(=O)R⁴, -NHSO₂R⁴, -(CH₂)ₘ-L¹-W, -O(CH₂)ₘ-L²-W₁ -C(O)(CH₂)ₘ-L³-W -OC(O) (CH₂)ₘ-L³-W, OC(O)₂(CH₂)ₘ-L³-W, - (C₅₋₆ heterocyclyl) -L⁴-W, SOCH₃, SOCN, SOCF₃, SO₂CH₃, SO₂CN, SO₂CF₃, SO₂NR⁵R⁶, C(=O)NR⁵R⁶, COCH₃, COCF₃, C(CN)₃ or either of the two specific ester groups as defined in claim 1;
R⁴, in each case, independently represents a hydrogen atom; an alkyl; alkenyl; alkynyl; heteroalkyl; cycloalkyl; heterocycloalkyl; aryl; heteroaryl; aralkyl; or heteroaralkyl group;
R⁵ and R⁶ each independently represents a hydrogen atom; or - (CH₂)ₘ-L⁵; or
R⁵ and R⁶ are taken together to form a 5- to 8-membered saturated, unsaturated or aromatic heterocyclic ring containing 1 to 4 N atoms or 1 to 3 heteroatoms selected from the group consisting of nitrogen, oxygen and sulphur, which heterocyclic ring may be unsubstituted or mono-, di or trisubstituted by halogen atom or L⁵; or R⁵ and R⁶ are taken together to form a 5- to 8-membered saturated, unsaturated or aromatic heterocyclic ring containing 1 to 3 heteroatoms selected from the group consisting of nitrogen, oxygen and sulphur, which is fused to one or two rings selected from the group consisting of cycloalkyl; heterocycloalkyl; alkylcycloalkyl; heteroalkylcycloalkyl; aryl; heteroaryl; aralkyl; and heteroaralkyl;
L¹, L², L³ and L⁴, in each case, independently represents a single bond, O, S, NH, SO, SO₂, -O(CH₂CH₂O)ₙ-, -(OCH₂CH₂)ₙ-, or - C(O)CH₂- (OCH₂CH₂)-NH]ₙ-;
L⁵ represents H; -(CH₂)ₚ-L⁶; - (CH₂)ₚ-OL⁶; a C₁₋₆ heteroalkyl; a cycloalkyl; a heterocycloalkyl; an alkylcycloalkyl; a heteroalkylcycloalkyl; an aryl; a heteroaryl; an aralkyl; or a heteroaralkyl group;
L⁶ represents a C₁₋₆ alkyl, C₃₋₆ alkenyl, or C₃₋₆ alkynyl group, in which 1 to 5 H atoms may, independently of each other, be replaced by halogen atom, CN, CF₃, NO₂, OR or NHR, and/or in which one or two non-adjacent CH₂ group(s) may be replaced by O, NH, S, SO, SO₂, or C₃₋₇ cycloalkyl;
W represents a hydrogen atom; a C₁₋₆ alkyl, C₃₋₆ alkenyl, or C₃₋₆ alkynyl group, in which 1 to 5 H atoms may, independently of each other, be replaced by halogen atom, CN, CF₃, NO₂, OR or NHR, and/or in which one or two non-adjacent CH₂ group(s) may be replaced by O, NH, S, SO, SO₂, or C₃₋₇ cycloalkyl; NR⁵R⁶, - NHC(=O)R⁴, -NHSO₂R⁴, -C(O)R⁵, -(CH)[(CH₂)ₘC(O)OR⁴]₂; or a 5, 6, or 7-membered saturated, unsaturated or aromatic carbocyclic or heterocyclic ring, which carbocyclic or heterocyclic ring may be substituted by 1, 2, or 3 R⁷;
R⁷, in each case, independently represents a halogen atom, an oxygen atom, a sulphur atom, CN, CF₃, NH₂, or NO₂;
X¹, X², and X³ each independently represents H or CH₃;
m each independently is 0, 1, 2, 3, or 4;
n each independently is an integer from 1 to 40; and
p each independently is 0, 1, 2 or 3.

Compounds are usually described herein using standard nomenclature or the definitions presented below. For compounds having asymmetric centers, it should be understood that (unless otherwise specified) all of the optical isomers and mixtures thereof are encompassed. Compounds with two or more asymmetric elements can also be present as mixtures of diastereomers. In addition, compounds with carbon-carbon double bonds may occur in Z- and E- forms, with all isomeric forms of the compounds being included in the present invention unless otherwise specified. Where a compound exists in various tautomeric forms, a recited compound is not limited to any one specific tautomer, but rather is intended to encompass all tautomeric forms. Recited compounds are further intended to encompass compounds in which one or more atoms are replaced with an isotope (*i.e.,* an atom having the same atomic number but a different mass number). By way of general example, and without limitation, isotopes of hydrogen include tritium and deuterium and isotopes of carbon include ¹¹C, ¹³C, and ¹⁴C.

Compounds according to the formulas provided herein, which have one or more stereogenic centers, have an enantiomeric excess of at least 50%. For example, such compounds may have an enantiomeric excess of at least 60%, 70%, 80%, 85%, 90%, 95%, or 98%. Some embodiments of the compounds have an enantiomeric excess of at least 99%. It will be apparent that single enantiomers (optically active forms) can be obtained by asymmetric synthesis, synthesis from optically pure precursors or by resolution of the racemates. Resolution of the racemates can be accomplished, for example, by conventional methods such as crystallization in the presence of a resolving agent, or chromatography, using, for example a chiral HPLC column.

Certain compounds are described herein using a general formula that includes variables, *e*. *g.* A, R¹, R², R³, R⁴, R⁵, R⁶, R⁷, L¹, L², L³, L⁴, L⁵, L⁶, W, X¹, X², X³, m, n, p, etc. Unless otherwise specified, each variable within such a formula is defined independently of any other variable, and any variable that occurs more than one time in a formula is defined independently at each occurrence. Thus, for example, if a group is shown to be substituted with 0-3 R³, the group may be unsubstituted or substituted with 1, 2 or 3 R³ groups, and R³ at each occurrence is selected independently from the definition of R³. Also, combinations of substituents and/or variables are permissible only if such combinations result in stable compounds, e. g., compounds that can be isolated, characterized and tested for biological activity.

As used herein a wording defining the limits of a range of length such as, e. g., "from 1 to 5" means any integer from 1 to 5, i. e. 1, 2, 3, 4 and 5. In other words, any range defined by two integers explicitly mentioned is meant to comprise and disclose any integer defining said limits and any integer comprised in said range. For example, the term "C₁₋₆" (or "C₁₋C₆") refers to 1 to 6, *i.e*. 1, 2, 3, 4, 5 or 6, carbon atoms.

A "pharmacologically acceptable salt" of a compound disclosed herein is an acid or base salt that is generally considered in the art to be suitable for use in contact with the tissues of human beings or animals without excessive toxicity or carcinogenicity, and preferably without irritation, allergic response, or other problem or complication. Such pharmaceutical salts include mineral and organic acid salts of basic residues such as amines, as well as alkali or organic salts of acidic residues such as carboxylic acids.

Suitable pharmaceutical salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-(CH₂)ₙ-COOH where n is any integer from 0 to -4 (*i.e*., 0, 1, 2, 3, or 4) and the like. Similarly, pharmaceutically acceptable cations include, but are not limited to sodium, potassium, calcium, aluminum, lithium and ammonium. Those of ordinary skill in the art will recognize further pharmacologically acceptable salts for the compounds provided herein. In general, a pharmacologically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two.

Generally, the use of nonaqueous media, such as ether, ethyl acetate, ethanol, isopropanol or acetonitrile, is preferred. It will be apparent that each compound of formula (I) may, but need not, be present as a hydrate, solvate or non-covalent complex. In addition, the various crystal forms and polymorphs are within the scope of the present invention. A "prodrug" is a compound that may not fully satisfy the structural requirements of the compounds provided herein, but is modified *in vivo,* following administration to a subject or patient, to produce a compound of formula I provided herein. For example, a prodrug may be an acylated derivative of a compound as provided herein. Prodrugs include compounds wherein hydroxy, carboxy, amine or sulfhydryl groups are bonded to any group that, when administered to a mammalian subject, cleaves to form a free hydroxy, carboxy, amino, or sulfhydryl group, respectively. Examples of prodrugs include, but are not limited to, acetate, formate, phosphate and benzoate derivatives of alcohol and amine functional groups within the compounds provided herein. Prodrugs of the compounds provided herein may be prepared by modifying functional groups present in the compounds in such a way that the modifications are cleaved *in vivo* to generate the parent compounds.

A "substituent," as used herein, refers to a molecular moiety that is covalently bonded to an atom within a molecule of interest. For example, a "ring substituent" may be a moiety such as a halogen, alkyl group, haloalkyl group, hydroxy, cyano, amino, nitro, mercapto, or other substituent described herein that is covalently bonded to an atom (preferably a carbon or nitrogen atom) that is a ring member. The term "substituted," as used herein, means that any one or more hydrogens on the designated atom is replaced with a selection from the indicated substituents, provided that the designated atom's normal valence is not exceeded, and that the substitution results in a stable compound, i.e. a compound that can be isolated, characterized and tested for biological activity. When a substituent is oxo (*i.e*., =O), then 2 hydrogens on the atom are replaced. An oxo group that is a substituent of an aromatic carbon atom results in a conversion of -CH- to -C(=O)- and a loss of aromaticity. For example a pyridyl group substituted by oxo is a pyridone.

As used herein, "comprising", "including", "containing", "characterized by", and grammatical equivalents thereof are inclusive or open-ended terms that do not exclude additional, unrecited elements or method steps. "Comprising", etc. is to be interpreted as including the more restrictive term "consisting of".

As used herein, "consisting of" excludes any element, step, or ingredient not specified in the claim.

When trade names are used herein, it is intended to independently include the trade name product formulation, the generic drug, and the active pharmaceutical ingredient(s) of the trade name product.

In general, unless defined otherwise, technical and scientific terms used herein have the same meaning as commonly understood by one of ordinary skill in the art to which this invention belongs, and are consistent with general textbooks and dictionaries.

The expression "optionally substituted" refers to groups in which one or more hydrogen atoms, e.g. 1 to 6 hydrogen atoms, have been replaced each independently of the others by fluorine, chlorine, bromine or iodine atoms; or by OH, =O, SH, =S, NH₂, =NH, CN or NO₂ groups. This expression refers furthermore to groups in which one or more hydrogen atoms have been replaced each independently of the others by unsubstituted C₁-C₆alkyl, (C₁-C₆) haloalkyl (e.g. a fluoromethyl, trifluoromethyl, chloromethyl, (1- or 2-)haloethyl (e.g. (1- or 2-) chloroethyl), or (2- or 3-) halopropyl (e.g. (2- or 3-) fluoropropyl) group), (C₁-C₆) hydroxyalkyl (e.g. a hydroxymethyl, (1- or 2-)hydroxyethyl, or (2- or 3-) hydroxypropyl group), unsubstituted C₂-C₆alkenyl, unsubstituted C₂-C₆alkynyl, unsubstituted C₁-C₆heteroalkyl, unsubstituted C₃-C₁₀cycloalkyl, unsubstituted C₂-C₉heterocycloalkyl, unsubstituted C₆-C₁₀aryl, unsubstituted C₁-C₉heteroaryl, unsubstituted C₇-C₁₂aralkyl or unsubstituted C₂-C₁₁heteroaralkyl groups.

The expression alkyl refers to a saturated, straight-chain or branched hydrocarbon group that contains from 1 to 20 carbon atoms, preferably from 1 to 12 carbon atoms, more preferably from 1 to 6 carbon atoms, for example a methyl, ethyl, propyl, iso-propyl, n-butyl, iso-butyl, *sec*-butyl, *tert*-butyl, n-pentyl, n-hexyl, 2,2-dimethylbutyl or n-octyl group. The alkyl groups may optionally be substituted.

The expression alkenyl refers to an at least partially unsaturated, straight-chain or branched hydrocarbon group that contains one or more double bond(s) and from 2 to 20 carbon atoms, preferably from 2 to 12 carbon atoms, more preferably from 2 to 6 carbon atoms, for example an ethenyl (vinyl), propenyl (allyl), iso-propenyl, butenyl, isoprenyl or hex-2-enyl group. Preferably, an alkenyl group has one or two, especially one, double bond(s).

The expression alkynyl refers to an at least partially unsaturated, straight-chain or branched hydrocarbon group that contains one or more triple bond(s) and from 2 to 20 carbon atoms, preferably from 2 to 12 carbon atoms, more preferably from 2 to 6, *e.g.* 2, 3 or 4, carbon atoms, for example an ethynyl (acetylenyl), propynyl, butynyl or propargyl group. Preferably, an alkynyl group has one or two, especially one, triple bond(s).

As used herein, the expression "heteroalkyl" also includes heteroalkenyl and heteroalkynyl, and accordingly refers to an alkyl, alkenyl or alkynyl (straigt chain or branched) group as defined above, in which one or more, preferably 1, 2, 3 or 4, carbon atoms have been replaced each independently of the others by an oxygen, nitrogen, phosphorus, boron, selenium, silicon or sulphur atom, preferably by an oxygen, sulphur or nitrogen atom, or by a SO or SO₂ group. As a result, the expression heteroalkyl also encompasses groups derived from a carboxylic acid, such as, for example, acyl, acylalkyl, alkoxycarbonyl, acyloxy, acyloxyalkyl, carboxyalkylamide or alkoxycarbonyloxy. Examples of heteroalkyl groups are alkylamino, dialkylamino, alkylaminoalkyl, dialkylaminoalkyl, acylalkyl, alkoxycarbonyl, alkylcarbamoyl, alkylamido, alkylcarbamoylalkyl, alkylamidoalkyl, alkylcarbamoyloxyalkyl, alkylureidoalkyl, alkoxycarbonyloxy, alkoxy, or alkoxyalkyl.

Further examples of heteroalkyl groups are groups of formulae: R^{a}-O-Y^{a}-, R^{a}-S-Y^{a}- , R^{a}-N(R^{b}) -Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b}) -CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a-}, R^{a}-N(R^{b}) -C(=NR^{d})-N(R^{c})-Y^{a}-, R^{a-}CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}- , R^{a}-S-CS-N(R^{b})-Y^{a}- , R^{a-}N(R^{b})-CS-S-Y^{a}-, R^{a}-S-CS-O-Y^{a}-, R^{a}-O-CS-S-Y^{a}-, wherein R^{a} being a hydrogen atom, a C₁-C₆ alkyl, a C₂-C₆ alkenyl or a C₂-C₆ alkynyl group; R^{b} being a hydrogen atom, a C₁-C₆ alkyl, a C₂-C₆ alkenyl or a C₂-C₆ alkynyl group; R^{c} being a hydrogen atom, a C₁-C₆ alkyl, a C₂-C₆ alkenyl or a C₂-C₆ alkynyl group; R^{d} being a hydrogen atom, a C₁-C₆ alkyl, a C₂-C₆ alkenyl or a C₂-C₆ alkynyl group and Y^{a} being a direct bond, a C₁-C₆ alkylene, a C₂-C₆ alkenylene or a C₂-C₆ alkynylene group. Specific examples of heteroalkyl groups include acyl, methoxy, trifluoromethoxy, ethoxy, n-propyloxy, isopropyloxy, *tert-*butyloxy, methoxymethyl, ethoxymethyl, methoxyethyl, methyl-amino, ethylamino, dimethylamino, diethylamino, isopropylethylamino, methylaminomethyl, ethylaminomethyl, diisopropylaminoethyl, dimethylaminomethyl, dimethylaminoethyl, acetyl, propionyl, butyryloxy, acetyloxy, methoxycarbonyl, ethoxycarbonyl, isobutyrylamino-methyl, N-ethyl-N-methylcarbamoyl, N-methylcarbamoyl, cyano, nitrile, isonitrile, thiocyanate, isocyanate, isothiocyanate and alkylnitrile.

The expression alkoxy refers to an alkyl group singular bonded to oxygen.

The expression alkylthio refers to an alkyl group singular bonded to sulfur.

The expression cycloalkyl refers to a saturated or partially unsaturated cyclic group that contains one or more rings (preferably 1 or 2), containing from 3 to 14 ring carbon atoms, preferably from 3 to 10 (more preferably 3, 4, 5, 6 or 7) ring carbon atoms. In an embodiment a partially unsaturated cyclic group has one, two or more double bonds, such as a cycloalkenyl group. Specific examples of a cycloalkyl group are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, cyclopentylcyclohexyl, and a cyclohex-2-enyl group.

The expression heterocycloalkyl refers to a cycloalkyl group as defined above in which one or more, preferably 1, 2 or 3, ring carbon atoms have been replaced each independently of the others by an oxygen, nitrogen, or sulphur atom (preferably oxygen or nitrogen), or by a SO or SO2 group. A heterocycloalkyl group has preferably 1 or 2 ring(s) containing from 3 to 10 (more preferably 3, 4, 5, 6 or 7) ring atoms. Examples are a aziridinyl, oxiranyl, thiiranyl, oxaziridinyl, dioxiranyl, azetidinyl, oxetanyl, thietanyl, diazetidinyl, dioxetanyl, dithietanyl, pyrrolidinyl, tetrahydrofuranyl, thiolanyl, phospholanyl, silolanyl, azolyl, thiazolyl, isothiazolyl, imidazolidinyl, pyrazolidinyl, oxazolidinyl, isoxazolidinyl, thiazolidinyl, isothiazolidinyl, dioxolanyl, dithiolanyl, piperazinyl, morpholinyl, thiopmorpholinyl, trioxanyl, azepanyl, oxepanyl, thiepanyl, homopiperazinyl, or urotropinyl group. Further examples are a 2-pyrazolinyl group, and also a lactam, a lactone and a cyclic imide. The heterocycloalkyl group can be optionally substituted, and may be saturated or mono-, di- or tri-unsaturated. As a result, a group derived from a carbohydrate or saccharide, such as furanoses or pentoses, e.g. arabinose, ribose, xylose, lyxose or desoxyribose, or pyranoses/ hexoses or derivatives thereof, e.g. allose, altrose, glucose, mannose, gulose, idose, galactose, talose, 6-carboxy-D-glucose, 6-carboxy-D-galactose, N-acetylchitosamine, glucosamine, N-acetylchondrosamin, fucose, rhamnose, chinovose, represents an optionally substituted heterocycloalkyl group.

The expression alkylcycloalkyl refers to a group containing both cycloalkyl and also an alkyl, alkenyl or alkynyl group in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two ring systems having from 3 to 10 (preferably 3, 4, 5, 6 or 7) carbon atoms, and one or two alkyl, alkenyl or alkynyl groups having 1 or 2 to 6 carbon atoms, the cyclic groups being optionally substituted.

The expression heteroalkylcycloalkyl refers to alkylcycloalkyl groups as defined above in which one or more (preferably 1, 2 or 3) carbon atoms have been replaced each independently of the others by an oxygen, nitrogen, silicon, selenium, phosphorus or sulphur atom (preferably oxygen, sulphur or nitrogen). A heteroalkylcycloalkyl group preferably contains 1 or 2 ring systems having from 3 to 10 (preferably 3, 4, 5, 6 or 7) ring atoms, and one or two alkyl, alkenyl, alkynyl or heteroalkyl groups having from 1 or 2 to 6 carbon atoms. Examples of such groups are alkylheterocycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being optionally substituted and saturated or mono-, di- or tri-unsaturated.

The expression aryl or Ar refers to an aromatic group that contains one or more rings containing from 6 to 14 ring carbon atoms, preferably from 6 to 10 (more preferably 6) ring carbon atoms. Examples are a phenyl, naphthyl, biphenyl, or indanyl group.

The expression heteroaryl refers to an aromatic group that contains one or more rings containing from 5 to 14 ring atoms, preferably from 5 to 10 (more preferably 5 or 6) ring atoms, and contains one or more (preferably 1, 2, 3 or 4) oxygen, nitrogen, phosphorus or sulphur ring atoms (preferably O, S or N). Examples are 2-pyridyl, 2-imidazolyl, 3-phenylpyrrolyl, thiazolyl, oxazolyl, triazolyl, tetrazolyl, isoxazolyl, indazolyl, indolyl, benzimidazolyl, pyridazinyl, quinolinyl, purinyl, carbazolyl, acridinyl, pyrimidyl, 2,3'-bifuryl, 3-pyrazolyl and isoquinolinyl.

The expression aralkyl refers to a group containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are toluene, xylene, mesitylene, styrene, benzyl chloride, o-fluorotoluene, 1H-indene, tetralin, dihydronaphthalene, indanone, phenylcyclopentyl, cumene, cyclohexylphenyl, fluorene and indan. An aralkyl group preferably contains one or two aromatic ring systems (1 or 2 rings) containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression heteroaralkyl refers to an aralkyl group as defined above in which one or more (preferably 1, 2, 3 or 4) carbon atoms have been replaced each independently of the others by an oxygen, nitrogen, silicon, selenium, phosphorus, boron or sulphur atom (preferably oxygen, sulphur or nitrogen), that is to say to groups containing both aryl or heteroaryl and also alkyl, alkenyl, alkynyl and/or heteroalkyl and/or cycloalkyl and/or heterocycloalkyl groups in accordance with the above definitions. A heteroaralkyl group preferably contains one or two aromatic ring systems (1 or 2 rings) containing from 5 or 6 to 10 ring carbon atoms and one or two alkyl, alkenyl and/or alkynyl groups containing 1 or 2 to 6 carbon atoms and/or a cycloalkyl group containing 5 or 6 ring carbon atoms, 1, 2, 3 or 4 of those carbon atoms having been replaced each independently of the others by oxygen, sulphur or nitrogen atoms. Examples of heteroaralkyl groups are arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroarylheterocycloalkyl, heteroarylheterocycloalkenyl, heteroarylalkylcycloalkyl, heteroarylalkylheterocycloalkenyl, heteroarylheteroalkylcycloalkyl, heteroarylheteroalkylcycloalkenyl, heteroalkylheteroarylalkyl and heteroarylheteroalkylheterocycloalkyl groups, the cyclic groups being saturated or mono-, di- or tri-unsaturated. Specific examples are a tetrahydroisoquinolinyl, benzoyl, 2- or 3-ethylindolyl, 4-methylpyridino, 2-, 3- or 4-methoxyphenyl, 4-ethoxyphenyl, 2-, 3- or 4-carboxyphenylalkyl group.

The expression "halogen" or "halogen atom" as preferably used herein means fluorine, chlorine, bromine, or iodine.

The expression "carbocyclic ring" as preferably used herein means a cycloalkyl or aryl group as defined above.

The expression "heterocyclic ring" as preferably used herein means a heterocycloalkyl or heteroaryl group as defined above. The term "heterocyclyl" means a heterocyclic ring from which a hydrogen atom has been removed from any ring atom to allow formation of a covalent bond between the heterocyclyl group and L⁴.

The activity and more specifically the bioactivity of the compounds according to the present invention can be assessed using appropriate assays known to those skilled in the art, e.g. *in vitro* or *in vivo* assays described in the above prior art references or those presented in the Examples below.

In the compound according to the present invention, or a pharmacologically acceptable salt thereof, R¹, R² or R³, especially R², can represent a group selected from the groups: 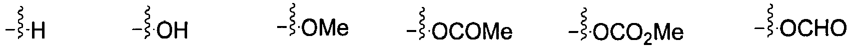 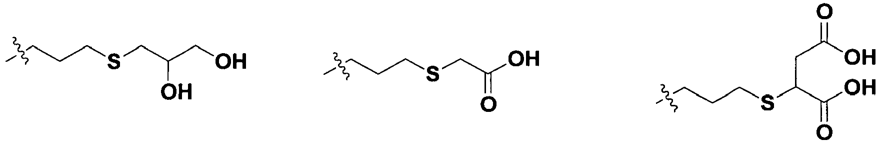 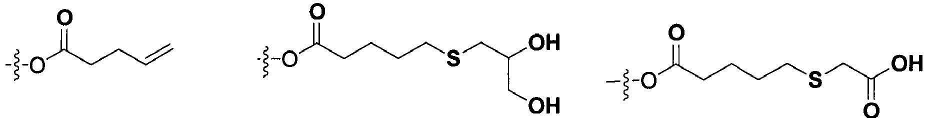 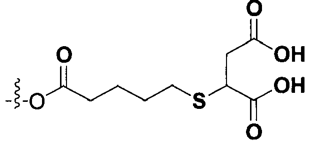 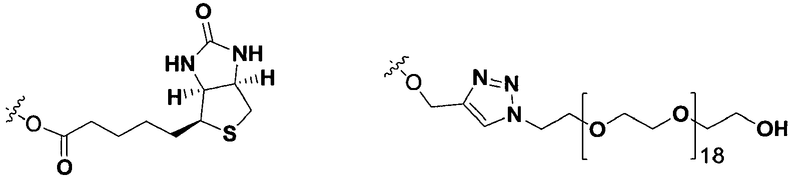 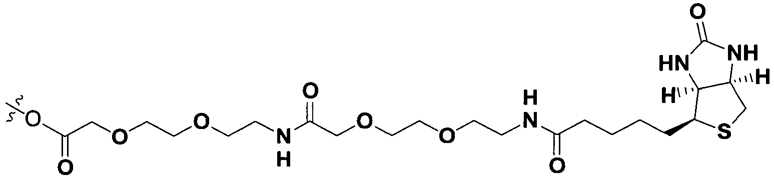 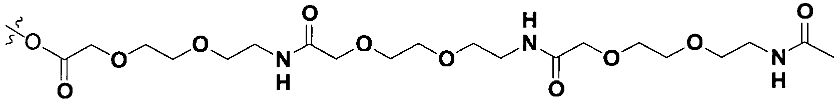 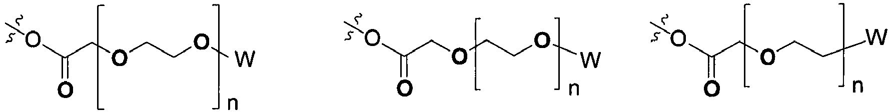 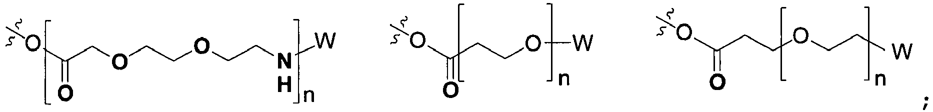 wherein n and W are defined as above.

Preferably, in the compound according to the invention, or a pharmacologically acceptable salt thereof, R¹ can represent OH or OMe.

In the compound according to the invention, or a pharmacologically acceptable salt thereof, R³ can preferably represent a hydrogen atom or a hydroxyl group.

Also preferred is a compound according to the invention, or a pharmacologically acceptable salt thereof, wherein R¹ represents OH or OMe; and R³ represents a hydrogen atom or a hydroxyl group.

Further preferred is a compound according to the invention, or a pharmacologically acceptable salt thereof, wherein A represents a group of the formula:

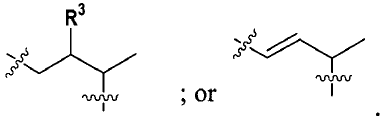

Preferably, in the compound according to the invention, or a pharmacologically acceptable salt thereof, X¹ can be CH₃ and X² can be H.

The compound of the invention, or a pharmacologically acceptable salt thereof, may be represented by one of the following formulas: 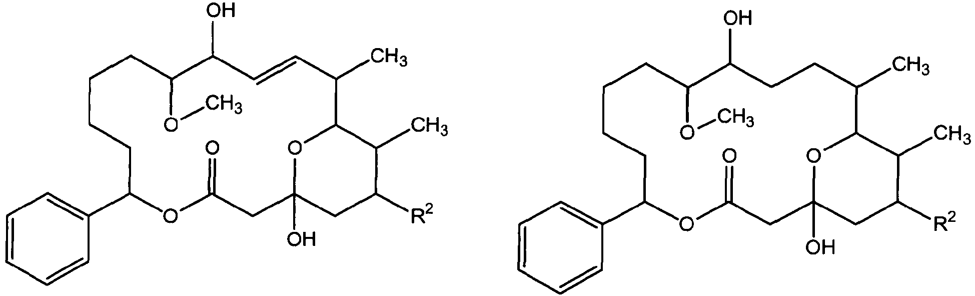 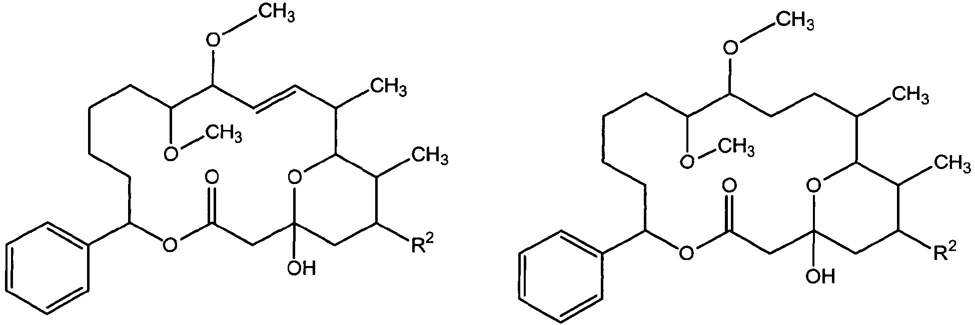 ; or 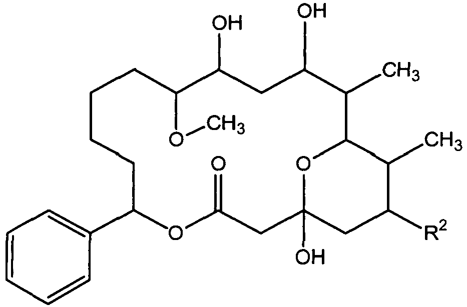 wherein R² is defined as above.

Preferably, the compound of formula (I), or a pharmacologically acceptable salt thereof, can be selected from the group consisting of:

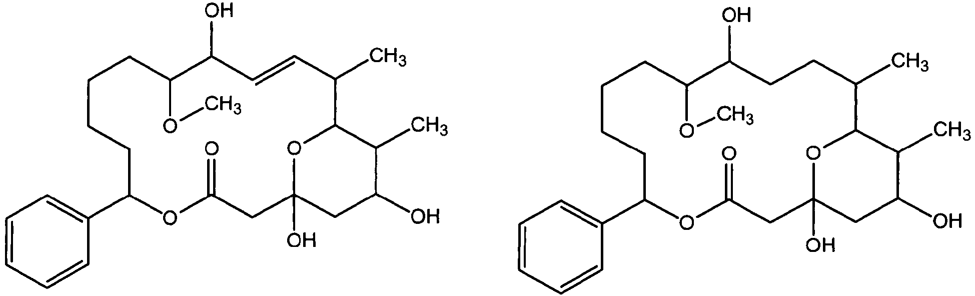

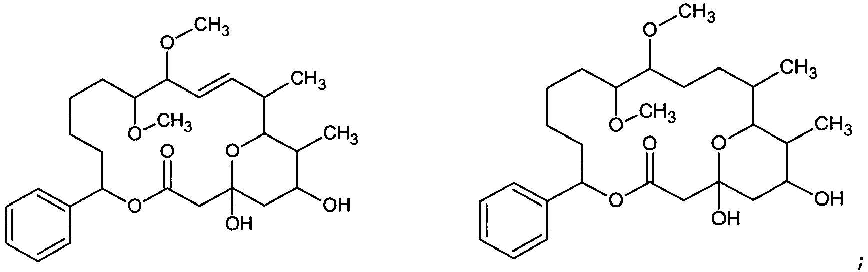

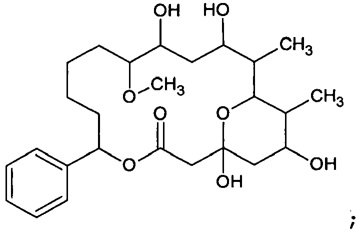

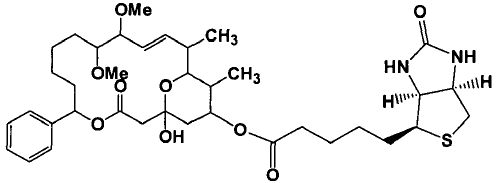

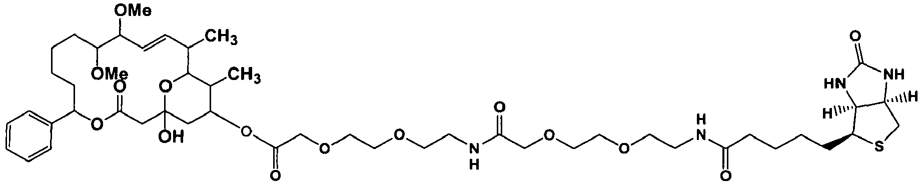

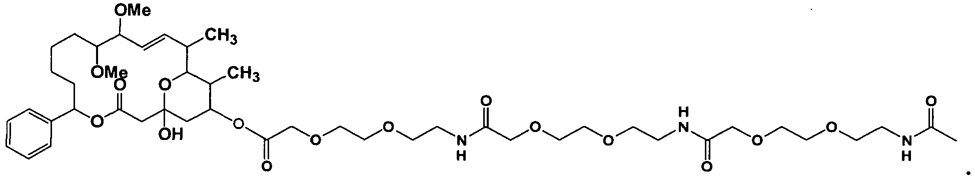

In an alternative preferred embodiment of the compound according to the invention, or a pharmacologically acceptable salt thereof, group A can represent a group of the formula:

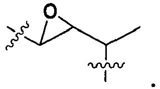

Further preferred is a compound according to the invention, or a pharmacologically acceptable salt thereof, wherein group A represents a group of the formula: 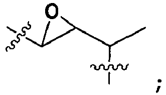 and
wherein R¹ can represent OH or OMe, or X¹ is CH₃ and X² is H; or a combination thereof; and R² and X³ are as defined above.

A compound of formula (I) can be obtained by chemical synthesis in a number of ways well known to one skilled in the art of organic synthesis using usual chemical reaction and synthesis methods.

The inventors established that targeting of a key enzyme in the regulation of mitochondrial fatty acid oxidation as well as of fatty acid synthesis, acetyl-CoA decarboxylase (ACC), inhibits the formation of Thl7 cells. In particular, the inventors established that the compounds of the invention inhibit ACC.

The inventors further established that addition of a compound of the invention to naive mouse CD4+ T cells decreases the percentage of IL-17A producing T-cells in a dose dependent manner.

The inventors also showed that treatment of mice with an ACC inhibitor according to the invention inhibited Th17 cell differentiation and delayed the onset and reduced the severity of autoimmune disease. In particular, the inventors demonstrate a positive effect of a compound of the invention in a mouse model of experimental autoimmune encephalomyelitis (EAE). More specifically, the inventors observed a positive effect on clinical score and a reduction of pro-inflammatory Th17 cells in the spinal cords of mice treated with a compound of the invention.

These surprising and unexpected results allow a therapeutic, preventive and/or curative role to be conceived for the compounds of the invention in the treatment or prevention of Th17-associated inflammatory diseases; and/or Thl7-associated autoimmune diseases. A therapeutic, preventive and/or curative role for the compounds of the invention may also be conceived in the treatment or prevention of viral infections.

Accordingly, the present invention is also directed to a novel inhibitor of acetyl-coenzyme A carboxylase (ACC inhibitor). By ACC inhibitor is meant any chemical or biological, natural or synthetic molecule, any composition which, whatever the mechanism, causes after administration a reduction, or even a complete inhibition, of the activity of ACC1 and/or ACC2. The ACC inhibitor may either bind reversible or irreversible to its substrate. Reversible ACC inhibitors comprise competitive inhibitors, non-competitive inhibitors, mixed-type inhibitors, uncompetitive inhibitors, slow-binding or tight-binding inhibitors, transition state analogs and multisubstrate analogs.

As indicated in the background section above, numerous ACC inhibitors are known and have been reported in the art, and by way of example those disclosed in the references cited above can be mentioned, including 1S,2S,3E,5R,6S,11S,14S,15R,16R,17S,18S)-15,17-dihydroxy-5,6,16-trimethoxy-2,14,18-trimethyl-11-phenyl-12,19-dioxabicyclo[13.3.1]nonadec-3-en-13-one (Soraphen A); 5-(tetradecyloxy)-2-furan-carboxylic acid (TOFA); CP-640186 [(3R)-1-[1-(anthracene-9-carbonyl)piperidin-4-yl]piperidin-3-yl]-morpholin-4-ylmethanone]; CP-610432 (S-1'-(anthracene-9-carbonyl) -N,N-diethyl-1,4'-bipiperidine-3-carboxamide); CP-610431 (R-1'-(anthracene-9-carbonyl)-N,N-diethyl-1,4'-bipiperidine-3-carboxamide); CP-497485 (1'-(anthracene-9-carbonyl) -N,N-diethyl-1,4'-bipiperidine-3-carboxamide); phenylmethyl 5-(1-{[(2-{[N-(2,4-dihydroxy-3,3-dimethylbutanyl)-5-(6-aminooctahydro-9H-purin-9-yl)-4-(hydroxyl-2-[(phosphonooxy)tetrahydrofuran-2-yl]methyl dihydrogen diphosphate-β-alanyl]amino}ethyl)thio]acetyl}-2-oxohexahydro-1H-thieno[3,4-d]imidazol-4-yl)pentanoate; 3,3-dimethylhexanoate, monoglyceride (AC-0417-9); MEDICA 16 (β,β,β',β'-tetramethylhexadecanoic acid); ESP-55016 (8-hydroxy-2,2,14,14-tetra-methylpentadecanediotic acid); and S2E ((+)-p-[1-p-tert-butylphenyl)-2-oxo-4-pyrrolidinyl]methoxybenzoic acid); 1'-N-Chloroacetamido-biotin, benzyl ester (CABI). According to the invention, it is also possible to use a combination of two or more ACC inhibitors in the compositions of the invention.

The compound of formula (I), its pharmacologically acceptable salts, solvates or hydrates and also formulations and pharmaceutical compositions which contain the same for therapeutic use are within the scope of the present invention. The present invention also relates to a compound or a pharmaceutical composition of the invention for use as a medicament. The present invention also relates to the use of a compound of formula (I) as active ingredient in the preparation or manufacture of a medicament.

A pharmaceutical composition according to the present invention comprises at least one compound of formula (I) and, optionally, one or more carrier substance(s), excipient(s) and/or adjuvant(s).

The term "at least one" as used herein is intended to mean one, 2, or more of the respective item or subject. Pharmaceutical compositions may additionally comprise, for example, one or more of water, buffers (*e.g*., neutral buffered saline or phosphate buffered saline), ethanol, mineral oil, vegetable oil, dimethylsulfoxide, carbohydrates (*e*.*g*., glucose, mannose, sucrose or dextrans), mannitol, proteins, adjuvants, polypeptides or amino acids such as glycine, antioxidants, chelating agents such as EDTA or glutathione and/or preservatives. Furthermore, one or more other active ingredients may (but need not) be included in the pharmaceutical compositions provided herein. For instance, the compounds of the invention may advantageously be employed in combination with an antibiotic or antifungal agent, an antiviral agent, an anti histamine, a non-steroidal antiinflammatory drug, a disease modifying anti-rheumatic drug, another cytostatic drug, a drug with smooth muscle activity modulatory activity or mixtures of the aforementioned.

Pharmaceutical compositions may be formulated for any appropriate route of administration, including, for example, topical such as, *e.g.,* transdermal or ocular, oral, buccal, nasal, vaginal, rectal or parenteral administration. The term parenteral as used herein includes subcutaneous, intradermal, intravascular such as, *e.g*., intravenous, intramuscular, spinal, intracranial, intrathecal, intraocular, periocular, intraorbital, intrasynovial and intraperitoneal injection, as well as any similar injection or infusion technique. Within the invention, compositions provided herein may be formulated as a lyophilizate. Formulation for topical administration may be preferred for certain conditions such as, *e.g.,* in the treatment of skin conditions such as burns or itch.

Carrier substances are, for example, cyclodextrins such as hydroxypropyl β-cyclodextrin, micelles or liposomes, excipients and/or adjuvants. Customary excipients include, for example, inert diluents such as, *e.g.,* calcium carbonate, sodium carbonate, lactose, calcium phosphate or sodium phosphate, granulating and disintegrating agents such as, *e.g.,* corn starch or alginic acid, binding agents such as, *e.g*., starch, gelatin or acacia, and lubricating agents such as, *e.g*., magnesium stearate or stearic acid. Examples of adjuvants are aluminum hydroxide, aluminum phosphate, calcium phosphate hydroxide, paraffin oil, squalene, thimerosal, detergents, Freund's complete adjuvant, or Freund's incomplete adjuvant.

The invention further relates to a combination preparation containing at least one compound according to the invention and at least one further active pharmaceutical ingredient.

Preferably, in the combination preparation of the invention the further active pharmaceutical ingredient is selected from (3*S,*6*R,*7*E,*9*R,*10*R,*12*R,*14*S,*15*E,*17*E,*19*E,*21*S,*23*S,* 26*R*,27*R*,34a*S*)-9,10,12,13,14,21,22,23,24,25,26,27,32,33,34,34a-hexadecahydro-9,27-dihydroxy-3-[(1*R*)-2-[(1*S*,3*R*,4*R*)-4-hydroxy-3-methoxycyclohexyl]-1-methylethyl]-10,21-dimethoxy-6,8,12,14,20,26-hexamethyl-23,27-epoxy-3*H*-pyrido[2,1-c][1,4]-oxaazacyclohentriacontine-1,5,11,28,29(4*H*,6*H*,31*H*)-pentone (Rapamycin); AP-23481, (1*R,*2*R,*4*S*)*-4-*{(2*R*)*-*2*-*[(3*S,*6*R,*7*E,*9*R,*10*R,* 12*R*,14*S*,15*E*,17*E*,19*E*,21*S*,23*S*,26*R*,27*R*,34a*S*)-9,27-dihydroxy-10,21-dimethoxy-6,8,12,14,20,26-hexamethyl-1,5,11,28,29-pentaoxo-1,4,5,6,9,10,11,12,13,14,21,22,23,24,25,26,27,28,29, 31,32,33,34,34a-tetracosahydro-3*H*-23,27-epoxypyrido[2,1-*c*] [1,4]oxazacyclohentriacontin-3-yl]propyl}-2-methoxycyclohexyl 3-hydroxy-2-(hydroxymethyl)-2-methylpropanoate (Temsirolimus), dihydroxy-12-[(2*R*)-1-[(1*S*,3*R*,4*R*)-4-(2-hydroxyethoxy)-3-methoxycyclohexyl]propan-2-yl]-19,30-dimethoxy-15,17,21,23,29,35-hexamethyl-11,36-dioxa-4-azatricyclo[30.3.1.0^{4,9}]hexatriaconta-16,24,26,28-tetraene-2,3,10,14,20-pentone (Everolimus) ; and (1*R*,2*R*,4*S*)-4-[(2*R*)-2-[(1*R,*9*S,*12*S,*15*R,*16*E,*18*R,*19*R,*21*R,*23*S,* 24*E*,26*E*,28*Z*,30*S*,32*S*,35*R*)-1,18-dihydroxy-19,30-dimethoxy-15,17,21,23,29,35-hexamethyl-2,3,10,14,20-pentaoxo-11,36-dioxa-4-azatricyclo[30.3.1.0^{4,9}]hexa-triaconta-16,24,26,28-tetraen-12-yl]propyl]-2-methoxycyclohexyl dimethylphosphinate (Ridaforolimus); antiviral drugs; or combinations thereof.

Antiviral drugs include, for example, Abacavir, Aciclovir, Acyclovir, Adefovir, Amantadine, Amprenavir, Ampligen, Arbidol, Atazanavir, Atripla, Balavir, Boceprevirertet, Cidofovir, Combivir, Dolutegravir, Darunavir, Delavirdine, Didanosine, Docosanol, Edoxudine, Efavirenz, Emtricitabine, Enfuvirtide, Entecavir, entry inhibitors, Famciclovir, Fomivirsen, Fosamprenavir, Foscarnet, Fosfonet, fusion inhibitor, Ganciclovir, Ibacitabine, Imunovir, Idoxuridine, Imiquimod, Indinavir, Inosine, integrase inhibitor, Interferon type III, Interferon type II, Interferon type I, Interferon, Lamivudine, Lopinavir, Loviride, Maraviroc, Moroxydine, Methisazone, Nelfinavir, Nevirapine, Nexavir, nucleoside analogues, Oseltamivir, Peginterferon alfa-2a, Penciclovir, Peramivir, Pleconaril, Podophyllotoxin, protease inhibitor, Raltegravir, reverse transcriptase inhibitor, Ribavirin, Rimantadine, Ritonavir, Pyramidine, Saquinavir, Sofosbuvir, Stavudine, synergistic enhancer (antiretroviral), Tea tree oil, Telaprevir, Tenofovir, Tenofovir disoproxil, Tipranavir, Trifluridine, Trizivir, Tromantadine, Truvada, Traporved, Valaciclovir, Valganciclovir, Vicriviroc, Vidarabine, Viramidine, Zalcitabine, Zanamivir, and Zidovudine.

The compound according to the invention as well as the pharmaceutical composition or the combination preparation according to the invention can be used as a medicament. In particular, the compound according to the invention, the pharmaceutical composition according to the invention, or the combination preparation according to the invention can be used in the treatment or prophylaxis of a Th17-associated inflammatory and/or autoimmune disease. Alternatively, the compound according to the invention, the pharmaceutical composition according to the invention, or the combination preparation according to the invention can be used in the treatment or prophylaxis of a viral infection.

By "treatment or prophylaxis" is preferably meant a reduction, alleviation or complete inhibiton of a viral infection, or a Th17-associated inflammatory and/or autoimmune disease, in a subject to thereby cure or prevent the symptoms associated with the viral infection and Th17-associated inflammatory and/or autoimmune disease, respectively.

As used herein, the term "subject" refers to a mammal, and preferably the "subject" is a human.

Preferably, the Th17-associated inflammatory and/or autoimmune disease is selected from: rheumatoid arthritis, psoriasis, systemic sclerosis, systemic lupus erythematosus, asthma, atopic dermatitis, contact hypersensitivity, multiple sclerosis, autoimmune myocarditis, type I diabetes, autoimmune thyroiditis, inflammatory bowel disease, Crohn's disease, ulcerative colitis, and transplant rejection. More preferably, the Th17-associated inflammatory and/or autoimmune disease is selected from: rheumatoid arthritis, psoriasis, asthma, multiple sclerosis, inflammatory bowel disease, Crohn's disease, and ulcerative colitis.

For the treatment or prophylaxis of a Th17-associated inflammatory and/or autoimmune disease, the dose of the biologically active compound according to the invention may vary within wide limits and may be adjusted to individual requirements. Active compounds according to the present invention are generally administered in a therapeutically effective amount. Preferred doses range from about 0.1 mg to about 140 mg per kilogram of body weight per day, and/or about 0.5 mg to about 7 g per patient per day. The daily dose may be administered as a single dose or in a plurality of doses. The amount of active ingredient that may be combined with the carrier materials to produce a single dosage form will vary depending upon the host treated and the particular mode of administration. Dosage unit forms will generally contain between from about 1 mg to about 500 mg of an active ingredient.

The viral infection may be caused by any virus, including enveloped viruses, non-enveloped viruses, DNA viruses and RNA viruses. Preferably, the viral infection is caused by a positive-strand RNA virus, a DNA virus or a retrovirus.

Positive-strand RNA viruses include, for example, Group IV-positive-sense ssRNA viruses, including the Togaviridae family comprising Rubella virus, Ross River virus, Sindbis virus, and Chikungunya virus; and the *Flaviviridae* family comprising the genera: Genus Flavi, including the species Dengue Virus 1-4; West Nile Virus; Yellow Fever Virus; Tick-borne Encephalitis Virus; Japanese Encephalitis Virus; St. Louis Encephalitis Virus; Murray Valley Encephalitis Virus; Kunjin Encephalitis Virus; Rocio Encephalitis Virus; Russian Spring Summer Encephalitis Virus; Negeishi Virus; Kyasanur Forest Virus; Omsk Hemorrhagic Fever Virus; Powassan Virus; Louping I11 Virus; Rio Bravo Virus 1-7; Tyuleniy Virus; Ntaya Virus; Uganda S Virus/Zika Virus; and Modoc Virus; the genus *Hepaci,* including the species Hepatitis C Virus (HCV) and Hepatitis G Virus; and the genus *Pesti* which contains viruses infecting non-human mammals, e. g. Bovine virus diarrhea 1-3. Major diseases caused by the *Flaviviridae* family include: Hepatitis C Virus (HCV) infection; Dengue fever; encephalitis; and hemorrhagic fever; and a major disease caused by the Togaviridae family is, for example, chikungunya.

DNA viruses include, for example, group I dsDNA viruses, including the order *Herpesvirales,* which *inter alia* includes the Herpesviridae family comprising human herpesviruses, and chicken pox/Varicella Zoster virus; the families *Adenoviridae, Asfarviridae, Iridoviridae, Papillomaviridae, Polyomaviridae* and *Poxviridae.*

Retroviruses include, for example, the Retroviridae family, including human immunodeficiency virus (HIV); and the Hepadnaviridae family, including Hepatitis B virus.

Preferably, the viral infection is caused by a virus selected from the group: HCV, Dengue viruses, West Nile virus, chikungunya virus, herpesviruses, and HIV.

For the treatment or prophylaxis of a viral infection, the dose of the biologically active compound according to the invention may vary within wide limits and may be adjusted to individual requirements. In any event, active compounds according to the present invention are generally administered in a therapeutically effective amount, and preferred doses as well as routes of administration correspond to those indicated above in relation to a Thl7-associated inflammatory and/or autoimmune disease.

According to the invention, the compound of the invention can be used in the pharmaceutical preparation in a quantity comprised between 0.01 mg and 2 g, preferably from 1 mg to 1 g, very preferably from 10 mg to 500 mg. More particularly, the compound of the invention can in particular be administered in doses comprised between 0.1 mg/kg and 500 mg/kg, preferably 1 mg/kg and 100 mg/kg, very preferably 10 mg/kg and 50 mg/kg.

It will be understood, however, that the specific dose level for any particular patient will depend upon a variety of factors including the activity of the specific compound employed, the age, body weight, general health, sex, diet, time of administration, route of administration, and rate of excretion, drug combination, *i.e*. other drugs being used to treat the patient, and the severity of the particular disease undergoing therapy.

The compound, the pharmaceutical composition, or the combination preparation of the invention is preferably administered to a patient orally or parenterally, and the compound of the invention is present within at least one body fluid or tissue of the subjected to be treated. Accordingly, the present invention further provides methods for preventing or treating patients suffering from a viral infection, a Th17-associated inflammatory disease, or a Th17-associated autoimmune disease.

As used herein, the term "treatment" encompasses both disease-modifying treatment and symptomatic treatment, either of which may be prophylactic, i.e., before the onset of symptoms, in order to prevent, delay or reduce the severity of symptoms, or therapeutic, *i.e.,* after the onset of symptoms, in order to reduce the severity and/or duration of symptoms.

Preferably, the method of preventing or treating a viral infection, a Th17-associated inflammatory disease, or a Th17-associated autoimmune disease comprises administering to a subject in need thereof an effective amount of at least one compound of the invention.

The method of preventing or treating a viral infection, a Thl7-associated inflammatory disease, or a Th17-associated autoimmune disease may, moreover, be characterized in that the viral infection is caused by a virus selected from the group: HCV, Dengue viruses, West Nile virus, chikungunya virus, herpesviruses, and HIV; or in that the Th17-associated inflammatory and/or autoimmune disease is selected from: rheumatoid arthritis, psoriasis, systemic sclerosis, systemic lupus erythematosus, asthma, atopic dermatitis, contact hypersensitivity, multiple sclerosis, autoimmune myocarditis, type I diabetes, autoimmune thyroiditis, inflammatory bowel disease, Crohn's disease, ulcerative colitis, and transplant rejection.

The method of preventing or treating a viral infection, a Th17-associated inflammatory disease, or a Th17-associated autoimmune disease may, moreover, be characterized in that the medicament of the invention is intended to be administered by oral route, by aerosol route or by injection.

Also the following method for producing a compound of formula (I) lies within the scope of the present invention. Since, the structural complexity of the compounds precludes facile total synthetic access, semisynthetic as well as biotechnological approaches are commonly pursued in pharmaceutical research and development. For instance, a compound of formula (I) can be prepared by a method comprising the steps of:
(a) fermenting *Sorangium cellulosum* (DSM 28093); and (b) separating and retaining the compound from the culture broth; wherein the compound is a compound according to the formulas shown above.

It is understood that the production of compounds of formula (I) is not limited to the use of the particular organism described herein, which is given for illustrative purpose only. The invention also includes the use of any mutants which are capable of producing a compound of formula (I) including natural mutants as well as artificial mutants, e.g. genetically manipulated mutants and the expression of the gene cluster responsible for biosynthesis in a producer strain or by heterologous expression in host strains.

A compound of formula (I) can be produced in liquid culture, by growing the respective microorganism in media containing one or several different carbon sources, and one or different nitrogen sources. Also salts are essential for growth and production. Suitable carbon sources are different mono-, di-, and polysaccharides like maltose, glucose or carbon from amino acids like peptones. Nitrogen sources are ammonium, nitrate, urea, chitin or nitrogen from amino acids. The following inorganic ions support the growth or are essential in synthetic media: Mg-ions, Ca-ions, Fe-ions, Mn-ions, Zn-ions, K-ions, sulfate-ions, Cl-ions, phosphate-ions.

Temperatures for growth and production are between 15°C to 40 °C, preferred temperatures are between 25 °C and 35 °C, especially at 30 °C. The pH of the culture solution is from 5 to 8, preferably a pH of 7.3 to 7.5.

A compound of formula (I) can also be obtained by chemical synthesis in a number of ways well known to one skilled in the art of organic synthesis using usual chemical reaction and synthesis methods.

It is especially preferred to combine the preferred embodiments of the present invention in any possible manner.

### Brief Description of the Figures

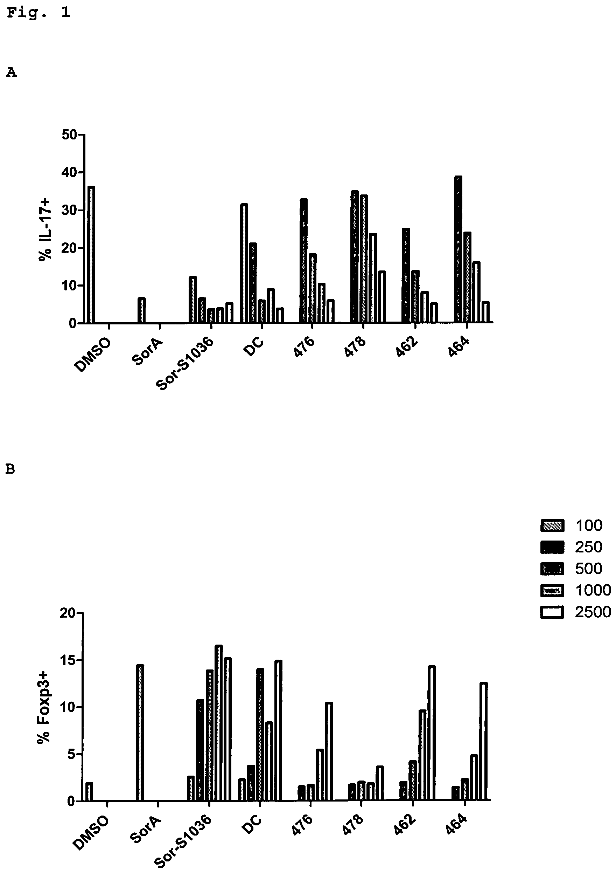

**Figure 1****:** Inhibition of Th17 and induction of Treg differentiation by the compounds of the present invention - Neosoraphene DC, 476, 478, 462 and 464, respectively - *in vitro.* **A:** Formation of IL17A+ cells within a live CD4+ T cell pool in the presence of varying concentrations of the compounds of the present invention compared to the known ACC inhibitor Soraphen A (SorA, 2^{nd} bar from the left) and DMSO (left bar). Mean of triplicates + s.d. **B:** Formation of Foxp3+ Treg cells within a live CD4+ T cell pool in the presence of varying concentrations of the compounds of the present invention compared to the known ACC inhibitor Soraphen A (SorA, 2^{nd} bar from the left) and DMSO (left bar). Mean of triplicates + s.d.

As can be taken from **Fig. 1****,** addition of the ACC inhibitor Soraphen A as well as of the compounds of the present invention decreases the percentage of IL-17A producing T-cells in a dose dependent manner (EC₅₀=40nM) and induces the reciprocal differentiation of Foxp3+ cells.

### Examples

### Materials and Methods

In general, standard bioassays and protocols were used in the following examples. Materials were normally purchased from commercial suppliers and used according to respective manuals supplied therewith.

Media for the cultivation of *Sorangium cellulosum* strain MSr1424 (DSM 28093):

| | |
|---|---|
| **CY+H-Medium:** | 0,15% casitone, 0,15% yeast extract (Marcor typ 9000), 0,1% glucose (monohydrate), 0,1% soy flour (degreased),0,4% starch (Cerestar), 0,1% CaCl₂ 2H₂O, 0,05% MgSO₄ x 7 H₂O, 0,0004% Na-Fe-EDTA, 50mM HEPES (11,8g/l); pH 7,2 |
| **M - Medium:** | 1,0% peptone (soy), 1,0% maltose monohydrate, 0,1% CaCl₂ x 2H₂O, 0,1% MgSO₄ x 4H₂O, 50mM HEPES (11,9g/l), 8mg/l Fe-EDTA; pH 7,2 |
| **P - Medium:** | 0,2% peptone (Marcor M), 0,8% starch (Cerestar), 0,4% Probion, 0,2% yeast extract (Marcor typ 9000), 0,1% CaCl₂ x 2H₂O, 0,1% MgSO₄ x 4H₂O, 100mM HEPES (23,8g/l), 8mg/l Fe-EDTA; pH 7,5 |
| **S-Medium:** | 0,4% soy flour (degreased), 0,2% glucose monohydrate, 0,8% starch (Cerestar), 0,1% CaCl₂ x 2H₂O, 0,1% MgSO₄ x 4H₂O, 50 mM HEPES (11,9 g/l), 8 mg/l Fe-EDTA; pH 7,4 |
| **Vitamin solution (Schlegel):** | 0,2% Biotin, 2,0% nicotinic acid, 1,0% Thiamin, 1,0% 4-Aminobenzoe acid, 0,5% Pantothenat, 5,0% Pyridoxamine, 2,0% Cyanocobalamine |
| **Vitamin B12 solution:** | 0,5 mg/mL |

- Add vitamins after autoclaving (100 µL in 100 mL medium)
- The vitamin solutions is sterilized by filtration and stored at 4°C.
- Adjust pH of medium with KOH.
- Cy+H-medium + Vit. + Vit.B12
- Cy+H-medium +5 mL P-medium + Vit. + Vit.B12
- S-medium + 5 mL P-medium + Vit. + Vit.B12
- M-medium + Vit. + Vit.B12

### In vitro Th17 differentiation

Single cell suspensions were prepared and pooled from the spleen, mesenteric and peripheral lymph nodes of adult C57BL/6 mice by filtering the crushed organs through a 70µm cell-strainer. Naïve CD4+CD25⁻CD62L+ T cells were subsequently isolated from these suspensions using the CD4+CD25⁻CD62L+ T cell isolation Kit II and AutoMACS according to the manufacturer's protocol (Miltenyi Biotec) or by FACS sorting. Purified T cells were cultured under classic Th17 inducing conditions (Bettelli et al., Nature 441 (7090): 235-8 (2006)). In detail, the cells were cultured in 96 well flat bottom plates (3 x 10⁵ cells per well), that were precoated with 10 µg/ml anti-CD3 Antibody (clone 145-2C11, BioXcell) in the presence of antibodies against CD28 (1 µg/ml, clone 37.51, BioXcell), IFN-γ (5 µg/ml, clone XMG1.2, BioXcell) and IL-4 (5 µg/ml, clone 11B11, BioXcell) as well as in the presence of TGFβ1 (2 ng/ml, R&D), IL-6 (5 ng/ml, R&D) and IL-1β (50 ng/ml, R&D) in IMDM Medium (supplemented with 10% FCS +500 U PenStrep + 50 µM β-Mercaptoethanol). To these cultures, increasing concentrations of ACC Inhibitors Soraphen A or 5-(Tetradecyloxy)-2-furoic acid (TOFA) (both dissolved in DMSO) were added on day 0 and cultured for up to 4 days in an cell culture incubator (37° C and 5% CO2). As control, cells were cultured in the presence of DMSO only.

### In vitro restimulation and Flow cytometry (FACS)

Were indicated, cells from Th17 cultures were restimulated with 100 ng/ml phorbol 12-myristate 13-acetate (Sigma) and 1 pg/ml Inonomycin (Sigma) for 4h and Brefeldin A (1:1000, Sigma) was added for the last 2h of restimulation. After restimulation, cells were harvested for flow cytometry.

For surface staining: fluorophore-labelled mAbs aginst CD3ε and CD4 were obtained from eBiosciences. For intracellular staining, cells were fixed with Fix/Perm (eBioscience) according to the manufacturer's protocol, and stained intracellular with fluorophore-labelled mAbs directed against IL-17A and Foxp3 (eBiosciences). Cells were run on a flow cytometer (CyAn, Beckman Coulter) and data was analysed on FlowJo (TreeStar) software.

To isolate naive CD4+CD25⁻CD62L+ T cells by FACS-sorting, lymphocyte suspensions were surface-stained with fluorophore-labelled mAbs aginst CD4, CD25 and CD62L (all from ebioscience) and live CD4+CD25⁻CD62L+ T cells were sorted to > 98% purity using a Moflo (Beckman Coulter) or FACS ARIAII (BD Biosciences) cell sorter.

### Example 1 Biosynthesis of Neosoraphens

**Cultivation of *Sorangium cellulosum* strain MSr1424 (DSM 28093):** 2 ml of cryo stock culture of strain MSr1424 (DSM28093) was carefully defrosted at room temperature and subsequently cultivated for 7 days at 30 °C in a 100 mL shake flask containing 20 mL of medium S supplemented with 5 mL medium P, vitamin solution (Schlegel) and vitamin B12. After 7 days of cultivation, the 20 mL seed culture was transferred in a 250 mL shake flask containing the same medium.

A 70L fermentation of strain MSr1424 (DSM 28093) was performed in the presence of 0.7L adsorber resin (Amberlite XAD-16). At the end of the fermentation (after 14 days) the adsorber resin was harvested by sieving with a process filter.

**Isolation of Neosoraphenes:** After sieving the adsorber resin (Amberlite XAD-16) from the culture broth, the adsorber resin was washed in an open glass column with 3L methanol/water 2:8 to remove polar substances. Subsequently the adsorber resin was extracted with 6L of methanol to yield raw Neosoraphens. After evaporation of the methanol, the residual waterphase was extracted three times with ethylacetate. The organic layer was dried, filtrated and concentrated in vacuo to give a crude extract of 9.8 g.

Initial separation was achieved in two steps by silica gel column chromatography on a Reveleris Flash chromatography System. Column: Reveleris Silica 80g, Grace; Elution solvent A: dichloromethane, solvent B: acetone, gradient: 11%B 3 min. isocratic, in 12 min. up to 20%B, in 6 min. to 29%B, isocratic for 13 min. at 29%B, in 2 min. to 100%B. Flow rate: 60 mL/min. Detection at 254 nm. Based on TLC analyses the chromatography gave 8 fractions. To separate the Neosoraphenes the fractions were further chromatographed on Nucleosil Si100, 7µm, 250x16 mm column (Macherey+Nagel) ; Solvent: t-Butylmethylether: n-Heptane 25:75 + 1% Methanol; Flow rate: 20 mL/min; Detection: 217 nm. In some cases a additional separation on Phenomenex Gemini column C18, 10 µm, 250x21 mm was used. Solvent A: acetonitrile : water = 2:8 + 0.1% acetic acid; Solvent B: acetonitrile : water = 4:6 + 0.1% acetic acid, Gradient: 80%B in 30 min. up to 100%B than 20 min. 100%B; Flow rate: 20 mL/min; Detection. 215 nm. Using this procedure, 95 mg Neosoraphen M462, 340 mg Neosoraphen M464, 600 mg Neosoraphen M476, 200 mg Neosoraphen M478, and 19 mg Neosoraphen M480 were obtained.

### Physico-chemical characterisation of Neosoraphen M462

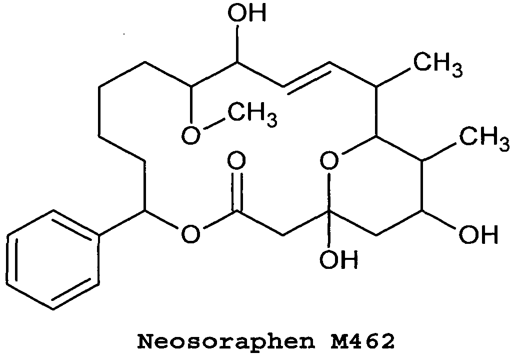

Formula: C₂₆H₃₈O₇
Molecular weight: 462.58
HR ESI MS: [M+Na]⁺ calcd.: 485.2509, found: 485.2504
IR (KBr): Y = 3423, 2920, 2850, 1736, 1436, 1222, 1164, 1091, 1053 cm⁻¹
UV (MeOH): λₘₐₓ (lgε) = endabsorption
HPLC: column: Waters ACQUITY UPLC BEH C18 Column, 2.1 x 50 mm, 1.7 µm; Solvent A: H2O + 0,1% HCOOH; Solvent B: ACN + 0,1% HCOOH; Gradient: 5%B-(0,5')-->5%B -(19,5')-->100% B-(10')--> 100% B; Flow: 0.6 ml/min. Detection: dioden array 200-600 nm; Retention time: 9.7min.

**Table 1. ¹H (600 MHZ) and ¹³C (150 MHz) NMR data of Neosoraphen M462 in CDCL₃**

| Proton | (ppm) | Multipl | J [Hz] | No. | C (ppm) |
|---|---|---|---|---|---|
| | | | | 1 | 167.5 |
| 2Ha | 2.66 | d | 12.8 | 2 | 47.6 |
| 2Hb | 2.55 | d | 12.8 | | |
| 3OH | 4.75 | s | | 3 | 96.2 |
| 4Ha | 1.83 | m | | 4 | 35.1 |
| 4Hb | 1.83 | m | | | |
| 5H | 3.89 | dq | 7, 3, 3, 2.9 | 5 | 71.3 |
| 5OH | 3.52 | d | 7.5 | | |
| 6H | 1.88 | m | | 6 | 35.8 |
| 6 Me | 0.90 | d | 7.0 | 6 Me | 10.3 |
| 7H | 3.82 | dd | 10.3, 2.2 | 7 | 72.6 |
| 8H | 2.40 | m | | 8 | 35.4 |
| 8 Me | 1.00 | d | 6.6 | 8 Me | 12.9 |
| 9H | 6.13 | dd | 16.1, 4.4 | 9 | 137.6 |
| 10H | 5.45 | ddd | 16.1, 9.0, 1.7 | 10 | 124.9 |
| 11H | 4.18 | td | 9.0, 8.6, 2.6 | 11 | 74.8 |
| 11OH | 2.47 | d | 8.4 | | |
| 12H | 3.34 | dt | 10.6, 3.3, 2.6 | 12 | 84.2 |
| 12OMe | 3.42 | s | | 12 OMe | 57.7 |
| 13Ha | 1.73 | m | | 13 | 29.7 |
| 13Hb | 1.25 | m | | | |
| 14Ha | 1.49 | m | | 14 | 23.3 |
| 14Hb | 1.14 | m | | | |
| 15Ha | 1.51 | m | | 15 | 25.9 |
| 15Hb | 1.46 | m | | | |
| 16Ha | 2.11 | ddt | 14.8, 11.4, 3.8 | 16 | 36.0 |
| 16Hb | 1.67 | | | | |
| 17H | 5.88 | dd | 11.6, 3.1 | 17 | 74.6 |
| | | | | 17' | 141.1 |
| 17H " * | 7.32 | m | | 17" * | 126.2 |
| 17H''' * | 7.30 | m | | 17"' * | 128.6 |
| 17H"" | 7.27 | m | | 17"" | 128.0 |

| | | | | | |
|---|---|---|---|---|---|
| *double | | | | | |

### Physico-chemical characterisation of Neosoraphen M464

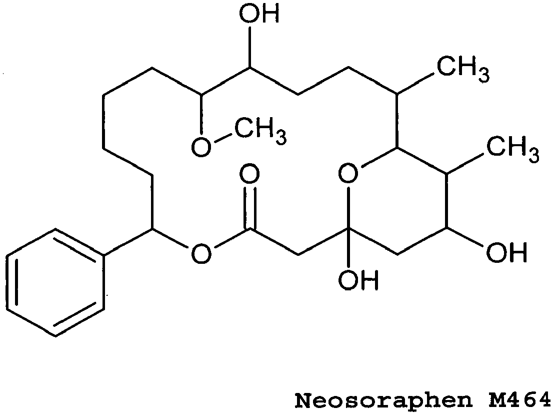

Formula: C₂₆H₄₀O₇
Molecular weight: 464.59
HR ESI MS: [M+Na]⁺ calcd.: 487.2666 found:487.2656
IR (KBr): γ = 3429, 2934, 1738, 1456, 1220, 1167, 1097, 1053 cm⁻¹
UV (MeOH) : λₘₐₓ (lgε) = endabsorption
HPLC: column: Waters ACQUITY UPLC BEH C18 Column, 2.1 x 50 mm, 1.7 µm; Solvent A: H₂O + 0,1% HCOOH; Solvent B: ACN + 0,1% HCOOH; Gradient: 5% B-(0,5')--> 5%B-(19,5')-->100% B --(10')-> 100%B; Flow: 0.6 ml/min; Detection: dioden array 200-600 nm; Retention time: 9.8 min.

**Table 2. ¹H (600 MHZ) and ¹³C (150 MHz) NMR data of Neosoraphen M464 in CDCL₃**

| Proton | (ppm) | Multipl. | Δ (Hz) | No. | C (ppm) |
|---|---|---|---|---|---|
| | | | | 1 | 168.0 |
| 2Ha | 2.67 | d | 13.9 | 2 | 47.1 |
| 2Hb | 2.54 | d | 13.6 | | |
| 3OH | 4.85 | s | | 3 | 96.2 |
| 4Ha | 1.97 | dd | 14.1, 3.1 | 4 | 35.0 |
| 4Hb | 1.76 | dd | 14.3, 2.2 | | |
| 5H | 3.95 | dd | 5.9, 2.9 | 5 | 71.8 |
| 5OH | 3.05 | d | 5.9 | | |
| 6H | 1.80 | m | | 6 | 35.3 |
| 6Me | 0.88 | d | 7.3 | 6Me | 10.2 |
| 7H | 3.93 | dd | 10.3, 2.2 | 7 | 69.0 |
| 8H | 1.85 | m | | 8 | 31.9 |
| 8Me | 0.85 | d | 7.0 | 8Me | 14.6 |
| 9Ha | 1.69 | m | | 9* | 28.1 |
| 9Hb | 1.56 | m | | | |
| 10Ha | 1.55 | m | | 10 | 24.8 |
| 10Hb | 1.46 | m | | | |
| 11H | 3.80 | m | | 11 | 70.9 |
| 11OH | 2.14 | d | 6.6 | | |
| 12H | 3.32 | td | 7.0, 7.0, 2.9 | 12 | 82.8 |
| 12OMe | 3.40 | s | | 12OMe | 57.4 |
| 13Ha | 1.73 | m | | 13* | 28.0 |
| 13Hb | 1.37 | m | | | |
| 14Ha | 1.51 | m | | 14 | 23.1 |
| 14Hb | 1.28 | m | | | |
| 15Ha | 1.58 | m | | 15 | 25.4 |
| 15Hb | 1.45 | m | | | |
| 16Ha | 2.07 | m | | 16 | 35.7 |
| 16Hb | 1.70 | m | | | |
| 17H | 5.93 | dd | 11.0, 3.7 | 17 | 74.6 |
| | | | | 17' | 141.1 |
| 17H" | 7.33 * | m | | 17" * | 126.4 |
| 17H'" | 7.32 * | m | | 17'" * | 128.5 |
| 17H"" | 7.27 | m | | 17"" | 128.0 |

| | | | | | |
|---|---|---|---|---|---|
| *double | | | | | |

### Physico-chemical characterisation of Neosoraphen M476

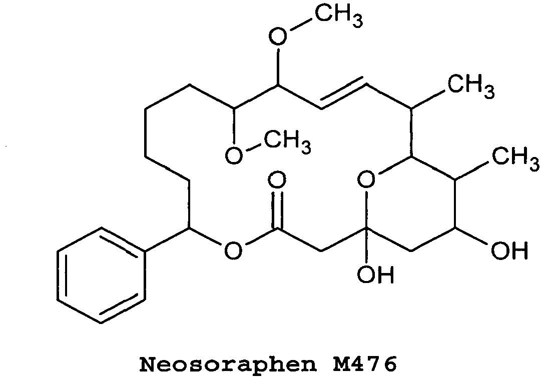

Formula: C₂₇H₄₀O₇
Molecular weight: 476.60
HR ESI MS: [M+Na]⁺ calcd.: 499.2666, found:499.2661
IR (KBr): γ = 3423, 2920, 2850, 1736, 1436, 1222, 1164, 1091, 1053 cm⁻¹
UV (MeOH): λₘₐₓ (lgε) = endabsorption
HPLC: column: Waters ACQUITY UPLC BEH C18 Column, 2.1 x 50 mm, 1.7 µm; Solvent A: H₂O + 0,1% HCOOH; Solvent B: ACN + 0,1% HCOOH; Gradient: 5%B-(0,5')-->5%B-(19,5')-->100% B --(10')--> 100%B; Flow: 0.6 ml/min; Detection: dioden array 200-600 nm; Retention time:10.3min.

**Table 3. ¹H (600 MHZ) and ¹³C (150 MHz) NMR data of Neosoraphen M476 in CDCL₃**

| Proton | (ppm) | Multipl. | Δ (Hz) | No. | C (ppm) |
|---|---|---|---|---|---|
| | | | | 1 | 167.7 |
| 2Ha | 2.67 | d | 12.5 | 2 | 47.7 |
| 2Hb | 2.54 | d | 12.5 | | |
| | | | | 3 | 96.2 |
| 3OH | 4.77 | s | | | |
| 4Ha | 1.83 | m | | 4 | 35.3 |
| 4Hb | 1.82 | m | | | |
| 5H | 3.90 | sbr | | 5 | 71.3 |
| 5OH | | d | | | |
| 6H | 1.88 | m | | 6 | 35.8 |
| 6Me | 0.91 | d | 7.3 | 6Me | 10.2 |
| 7H | 3.82 | dd | 10.3, 2.2 | 7 | 72.6 |
| 8H | 2.44 | m | | 8 | 35.3 |
| 8Me | 1.02 | d | 7.0 | 8Me | 12.7 |
| 9H | 6.20 | dd | 16.1, 4.4 | 9 | 140.1 |
| 10H | 5.44 | ddd | 16.1, 9.4, 1.3 | 10 | 122.5 |
| 11H | 3.68 | dd | 9.2, 1.8 | 11 | 84.8 |
| 11OMe | 3.29 | s | | 11OMe | 56.3 |
| 12H | 3.41 | m | | 12 | 83.1 |
| 12OMe | 3.43 | s | | 12OMe | 58.0 |
| 13Ha | 1.65 | m | | 13 | 30.6 |
| 13Hb | 1.28 | m | | | |
| 14Ha | 1.47 | m | | 14 | 23.4 |
| 14Hb | 1.11 | m | | | |
| 15Ha | 1.54 | m | | 15 | 25.9 |
| 15Hb | 1.47 | m | | | |
| 16Ha | 2.10 | m | | 16 | 35.9 |
| 16Hb | 1.67 | m | | | |
| 17H | 5.91 | dd | 11.7, 3.3 | 17 | 74.3 |
| | | | | 17' | 141.2 |
| 17H" * | 7.33 | m | | 17" * | 126.2 |
| 17H'" * | 7.32 | m | | 17"' * | 128.5 |
| 17H"" | 7.27 | m | | 17"" | 128.0 |

| | | | | | |
|---|---|---|---|---|---|
| *double | | | | | |

### Physico-chemical characterisation of Neosoraphen M478

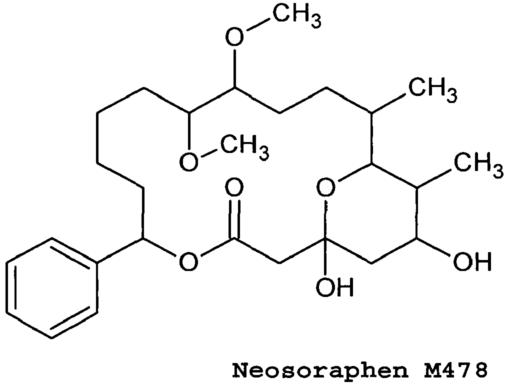

Formula: C₂₇H₄₂O₇
Molecular weight: 478.62
HR ESI MS: [M+Na]⁺ calcd.: 501.2823 found: 501.2820
IR (KBr): γ = 3423, 2920, 2850, 1736, 1436, 1222, 1164, 1091, 1053 cm⁻¹
UV (MeOH) : λₘₐₓ (lgε ) = endabsorption
HPLC: column: Waters ACQUITY UPLC BEH C18 Column, 2.1 x 50 mm, 1.7 µm; Solvent A: H₂O + 0,1% HCOOH; Solvent B: ACN + 0,1% HCOOH; Gradient: 5%B-(0,5')--> 5%B-(19,5')-->100% B --(10')--> 100%B; Flow: 0.6 ml/min; Detection: dioden array 200-600 nm; Retention time:11.6 min.

**Table 4. ¹H (600 MHZ) and ¹³C (150 MHz) NMR data of Neosoraphen M478 in CDCL₃**

| Proton | (ppm) | Multipl. | Δ (Hz) | No. | C (ppm) |
|---|---|---|---|---|---|
| | | | | 1 | 168.1 |
| 2Ha | 2.67 | d | 13.9 | 2 | 47.3 |
| 2Hb | 2.55 | d | 13.6 | | |
| | | | | 3 | 96.2 |
| 3OH | 4.70 | s | | | |
| 4Ha | 1.93 | m | | 4 | 35.1 |
| 4Hb | 1.79 | m | | | |
| 5H | 3.94 | sbr | | 5 | 71.7 |
| 5OH | 2.94 | d | 13.9 | | |
| 6H | 1.82 | m | | 6 | 35.4 |
| 6Me | 0.88 | d | 7.0 | 6Me | 10.2 |
| 7H | 3.93 | m | | 7 | 69.0 |
| 8H | 1.81 | m | | 8 | 32.3 |
| 8Me | 0.86 | d | 7.0 | 8Me | 14.3 |
| 9Ha | 1.73 | m | | 9 | 28.4 |
| 9Hb | 1.50 | m | | | |
| 10Ha | 1.55 | m | | 10 | 23.1 |
| 10Hb | 1.55 | m | | | |
| 11H | 3.35 | m | | 11 | 81.5 |
| 11OMe | 3.42 | s | | 11OMe | 57.7 |
| 12H | 3.40 | m | | 12 | 80.3 |
| 12OMe | 3.42 | s | | 12OMe | 57.6 |
| 13Ha | 1.73 | m | | 13 | 28.8 |
| 13Hb | 1.43 | m | | | |
| 14Ha | 1.55 | m | | 14 | 23.4 |
| 14Hb | 1.30 | m | | | |
| 15Ha | 1.54 | m | | 15 | 25.0 |
| 15Hb | 1.43 | m | | | |
| 16Ha | 2.06 | m | | 16 | 35.5 |
| 16Hb | 1.71 | m | | | |
| 17H | 5.92 | dd | 11.3, 4.4 | 17 | 74.8 |
| | | | | 17' | 141.0 |
| 17H" * | 7.33 | m | | 17" * | 126.5 |
| 17H" * | 7.32 | m | | 17"' * | 128.5 |
| 17H"" | 7.27 | m | | 17"" | 128.0 |

| | | | | | |
|---|---|---|---|---|---|
| *double | | | | | |

### Physico-chemical characterisation of Neosoraphen M480

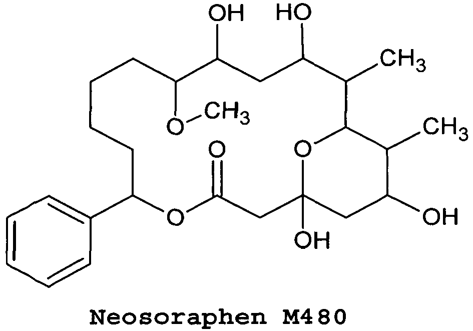

Formula: C₂₆H₄₀O₈
Molecular weight: 480.59
HR ESI MS: [M+Na]⁺ calcd.: 503.2615 found:503.2611
IR (KBr): γ = 3423, 2920, 2850, 1736, 1436, 1222, 1164, 1091, 1053 cm⁻¹
UV (MeOH): λₘₐₓ (lgε) = endabsorption
HPLC: column: Waters ACQUITY UPLC BEH C18 Column, 2.1 x 50 mm, 1.7 µm; Solvent A: H₂O + 0,1% HCOOH; Solvent B: ACN + 0,1% HCOOH; Gradient: 5%B-(0,5')--> 5%B-(19,5')-->100% B -(10')--> 100%B; Flow: 0.6 ml/min; Detection: dioden array 200-600 nm; Retention time: 8.9 min.

**Table 5. ¹H (600 MHZ) and ¹³C (150 MHz) NMR data of Neosoraphen M480 in CDCL₃**

| Proton | (ppm) | Multipl. | Δ (Hz) | No. | C (ppm) |
|---|---|---|---|---|---|
| | | | | 1 | 167.60 |
| 2Ha | 2.63 | d | 12.5 | 2 | 47.4 |
| 2Hb | 2.49 | d | 12.5 | | |
| | | | | 3 | 96.2 |
| 3 OH | 5.17 | s | | | |
| 4Ha | 1.82 | m | | 4 | 35.7 |
| 4Hb | 1.82 | m | | | |
| 5H | 4.02 | ddd | 2.9, 2.9, 2.9 | 5 | 71.9 |
| 5 OH | | | | | |
| 6H | 1.79 | m | | 6 | 35.5 |
| 6 Me | 0.91 | d | 7.0 | 6 Me | 10.2 |
| 7H | 3.98 | dd | 11.0, 2.6 | 7 | 68.1 |
| 8H | 2.05 | m | | 8 | 39.3 |
| 8 Me | 0.84 | d | 7.0 | 8 Me | 8.8 |
| 9H | 4.39 | ddd | 11.2, 4.2, 2.2 | 9 | 72.2 |
| 9 OH | 3.17 | sbr | | | |
| 10Ha | 1.67 | m | | 10 | 27.5 |
| 10Hb | 1.44 | m | | | |
| 11H | 4.09 | d | 9.2 | 11 | 73.7 |
| 11 OH | 3.08 | sbr | | | |
| 12H | 3.36 | ddd | 10.6, 3.0, 3.0 | 12 | 84.2 |
| 12 OMe | 3.43 | s | | 12 OMe | 57.8 |
| 13Ha | 1.84 | m | | 13 | 29.8 |
| 13Hb | 1.27 | m | | | |
| 14Ha | 1.49 | m | | 14 | 23.3 |
| 14Hb | 1.18 | m | | | |
| 15Ha | 1.64 | m | | 15 | 25.4 |
| 15Hb | 1.44 | m | | | |
| 16Ha | 2.11 | m | | 16 | 35.8 |
| 16Hb | 1.66 | m | | | |
| 17H | 6.02 | Dd | 11.4, 2.8 | 17 | 73.4 |
| | | | | 17' | 141.4 |
| 17H" * | 7.33 | M | | 17" * | 126.5 |
| 17H"' * | 7.32 | M | | 17"' * | 128.5 |
| 17H"" | 7.33 | M | | 17"" | 127.9 |

| | | | | | |
|---|---|---|---|---|---|
| *double | | | | | |

### Example 2 Chemical synthesis of 5-substituted Neosoraphens

A 5-substituted compound of formula (I) can be obtained by chemical synthesis in a number of ways well known to one skilled in the art of organic synthesis using usual chemical reaction and synthesis methods. For example, 5-substituted compounds of formula (I) can be obtained according to Reaction Scheme 1 shown below.

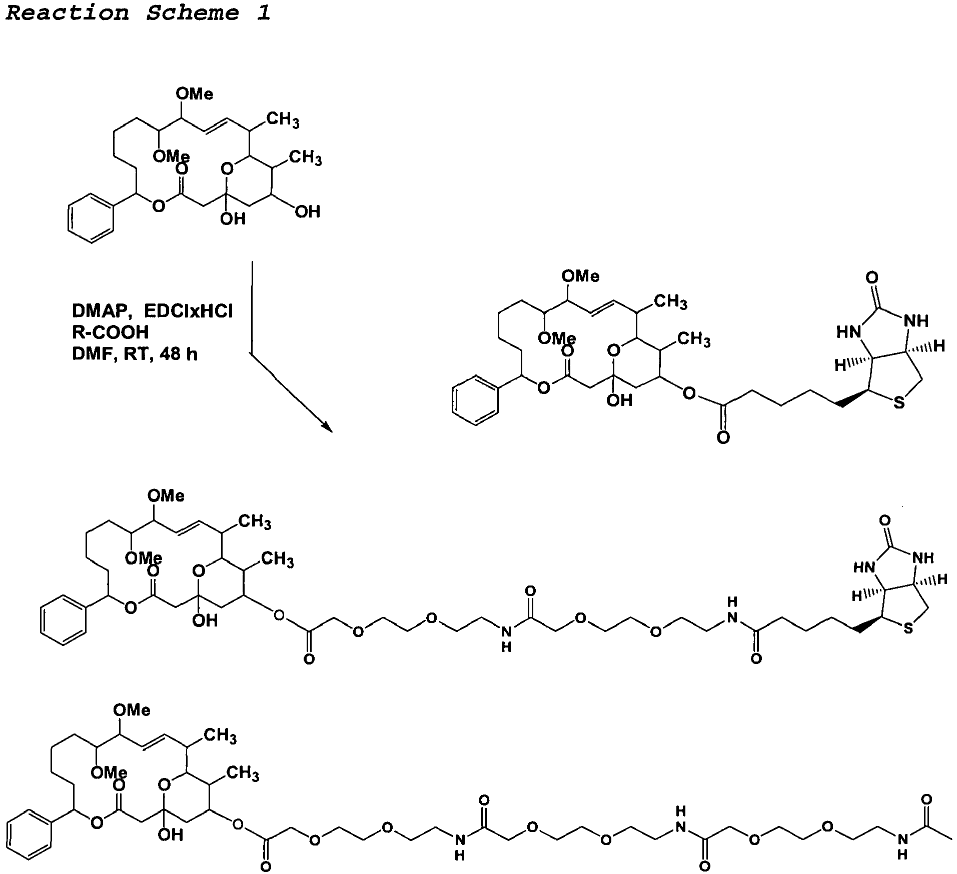

1 eq of biotinylated linker (IRIS Biotech), dissolved in DMF (∼ 0.1 M), and 0.2 equivalents of EDCI*HCl were stirred for ten min at room temperature. After this time 0.2 eq DMAP and after five min 0.11 eq of Neosoraphen M476 were added. After ca. 12 h 0.1 additional eq of EDCI*HCl and DMAP were added. The mixture was stirred at room temperature for eight additional hours before NH₄Cl solution was added for hydrolysis (2x volume of DMF) and the mixture was extracted with EtOAc. The organic phase was dried with MgSO₄, filtered and dried *in vacuo.* The crude product was purified by HPLC using a Waters auto purifier system equipped with a Waters XBridge column (prep C18, 5µm, 19 x 150 mm) applying a H₂O+0.1% HCO₂H(A) /MeOH+0.1% HCO₂H(B) gradient from 10 %B to 90 %B in 30 min to thereby yield 31 % of the 5-substituted Neosoraphen M476.

### Example 3: Chemical synthesis of a Neosoraphen M476-epoxide derivative

A Neosoraphen M476-epoxide derivative has been synthezised according to Reaction Scheme 2 shown below.

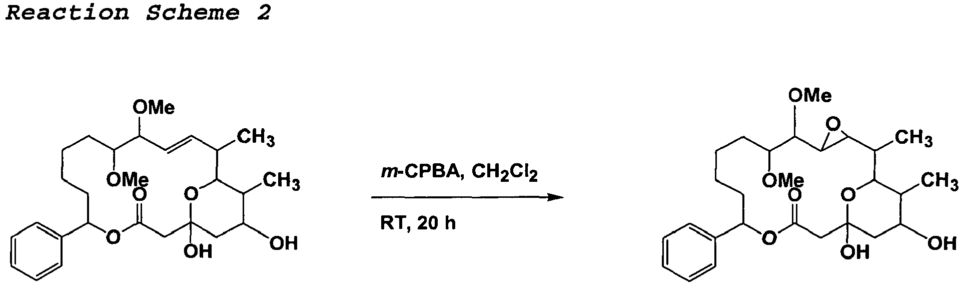

### Example 4: Inhibition of Th17 and induction of Treg differentiation (in vitro)

Naive mouse CD4⁺ T cells were cultured for 4 days under Thl7 conditions in the presence of varying concentrations of the compounds of the invention. As control, cells were cultured in the presence of DMSO only (negative control) or ACC inhibitor Soraphen A (positive control). After 4 days, cells were restimulated with PMA/Ionomycin for 4 h and Brefeldin A for 2h and further analysed by flow cytometry. The results are shown in **Fig. 1A** and **1B****.** These results show that addition of Soraphen A as well as of the compounds of the invention decreases the percentage of IL-17A producing T-cells in a dose dependent manner **(****Fig. 1A****;** EC₅₀= 40nM) and induces the reciprocal differentiation of Foxp3+ Treg cells **(****Fig. 1B****).** Thus, the results indicate that inhibition of ACC significantly lowers formation of pro-inflammatory Th17 cells and favours formation of Foxp3+ Tregs that are crucial for the induction and maintenance of self-tolerance and for the prevention of autoimmunity.

### Example 5: Inhibition of HCV infection (in vitro)

To assess the inhibitory effects of Neosoraphens on HCV infection, a HCV cell-culture (HCVcc) system^{[1-3]} was used. Unless otherwise stated, Huh7/Scr cells (provided by F. Chisari, The Scripps Research Institute, La Jolla, CA) which are highly permissive for HCV propagation *in vitro* were employed. The experiments were performed in the context of genotype 2a (Jc1 chimera^{[4]}) HCVcc virus. To facilitate the quantification of infection, the bicistronic Jc1 luciferase reporter construct, designated Luc-Jc1^{[5]}, was used. The commercial ACC inhibitor 5-(Tetradecyloxy)-2-furoic acid (TOFA), which has been previously described to inhibit HCV replication^{[61]}, was chosen for comparison of the antiviral activities of the Neosoraphens. The NS3-4A serine protease inhibitor VX-950^{[71]}, a known HCV replication inhibitor, was used as positive control. Briefly, Huh7/Scr cells were seeded at a density of 1.2 x 10⁴ cells/well in 96-well plates. One day later cells were pre-incubated for 1h at 37°C with the compounds and then inoculated with the virus and the compounds for 4h at 37°C. Finally, virus-containing media was replaced by a fresh media-compounds mix. Firefly luciferase activities were assayed 72h post infection with the Dual-Glo® Luciferase Assay System while cytotoxicity (viability) assays were carried out with the CytoTox-Glo™ Cytotoxicity Assay (both purchased from Promega Corporation, Madison, WI), according to manufacturer's instructions, using a plate luminometer FLUOstar OPTIMA (BMG LABTECH, Ortenberg, Germany). Mean relative light units (RLU) were plotted as percentage relative to control infections (solvent without compounds) for both infectivity and cell viability. Infections were carried out in triplicates and measured in duplicates (mean ± SEM; n=6). Half maximal effective concentration (EC₅₀) and half maximal cytotoxic concentration 50 (CC₅₀) were estimated by non-linear regression of log inhibitor vs. normalized response and used to calculate the Selectivity Index (SI) value. The results of the experiments are summarized in Table 6 below.

**Table 6. In vitro antiviral activity (EC₅₀, CC₅₀, SI).**

| **Compound** | **EC₅₀ (nM)** | **CC₅₀ (µM)** | **SI** |
|---|---|---|---|
| Neosoraphen M462 | 7.74 | 13.56 | 1752 |
| Neosoraphen M464 | 46.00 | 52.24 | 1136 |
| Neosoraphen M476 | 2.09 | 198.7 | 95072 |
| Neosoraphen M478 | 1.52 | 98.74 | 64961 |
| Neosoraphen M480 | 65.73 | 45.52 | 693 |
| TOFA | 1010 | 33.87 | 34 |
| VX-950 | 18.92 | 94.51 | 4995 |

### References

[1] Zhong J et al., PNAS 2005;102:9294-9299.
[2] Lindenbach BD et al., Science 2005;309:623-626.
[3] Wakita et al., Nature medicine 2005;11:791-796.
[4] Pietschmann T et al., PNAS 2006;103:7408-7413.
[5] Koutsoudakis G et al., J Virol. 2006;80:5308-5320.
[6] Kapadia SB, Chisari FV, PNAS 2005;102:2561-2566.
[7] Lin C, Kwong AD, Perni RB, Infectious disorders drug targets 2006;6:3-16.

The features of the present invention disclosed in the specification, the claims and/or the drawings may both separately and in any combination thereof be material for realizing the invention in various forms thereof. Especially preferred are combinations of preferred embodiments of the invention.

## Claims

1. A compound of the general formula (I): 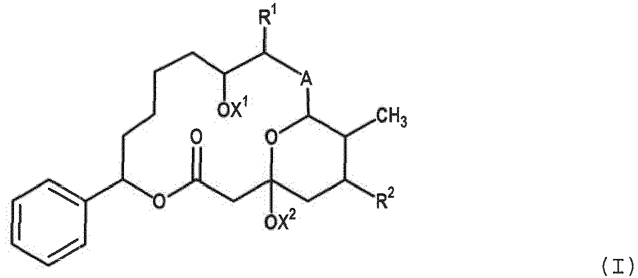 or a pharmacologically acceptable salt thereof, wherein
A represents a group of the formula: 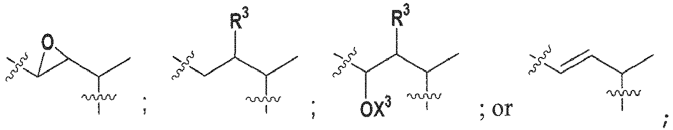 R¹, R² and R³, in each case, independently represents a hydrogen atom, a halogen atom, CN, CF₃, NO₂, NH₂,-NHC(=O)R⁴, -NHSO₂R⁴, -(CH₂)ₘ-L¹-W, -O(CH₂)ₘ-L²-W, -C(O)(CH₂)ₘ-L³-W, -OC(O)(CH₂)ₘ-L³-W, OC(O)₂(CH₂)ₘ-L³-W, -(C₅₋₆ heterocyclyl)-L⁴-W, SOCH₃, SOCN, SOCF₃, SO₂CH₃, SO₂CN, SO₂CF₃, SO₂NR⁵R⁶, C(=O)NR⁵R⁶, COCH₃, COCF₃, C(CN)₃, 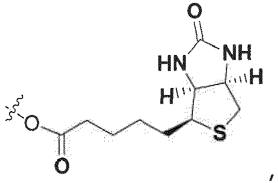 or 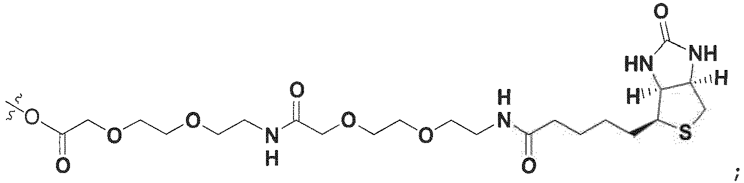
R⁴, in each case, independently represents a hydrogen atom; an alkyl; alkenyl; alkynyl; heteroalkyl; cycloalkyl; heterocycloalkyl; aryl; heteroaryl; aralkyl; or heteroaralkyl group;
R⁵ and R⁶ each independently represents a hydrogen atom; or - (CH₂)ₘ-L⁵; or
R⁵ and R⁶ are taken together to form a 5- to 8-membered saturated, unsaturated or aromatic heterocycle containing 1 to 4 N atoms or 1 to 3 heteroatoms selected from the group consisting of nitrogen, oxygen and sulphur, which heterocycle may be unsubstituted or mono-, di or trisubstituted by halogen atom or L⁵; or R⁵ and R⁶ are taken together to form a 5- to 8-membered saturated, unsaturated or aromatic heterocycle containing 1 to 3 heteroatoms selected from the group consisting of nitrogen, oxygen and sulphur, which is fused to one or two rings selected from the group consisting of cycloalkyl; heterocycloalkyl; alkylcycloalkyl; heteroalkylcycloalkyl; aryl; heteroaryl; aralkyl; and heteroaralkyl;
L¹, L², L³ and L⁴, in each case, independently represents a single bond, O, S, NH, SO, SO₂, -O(CH₂CH₂O)ₙ-, -(OCH₂CH₂)ₙ-, or-[C(O)CH₂-(OCH₂CH₂)₂-NH]ₙ-;
L⁵ represents H; -(CH₂)ₚ-L⁶; -(CH₂)ₚ-OL⁶; a C₁₋₆ heteroalkyl; a cycloalkyl; a heterocycloalkyl; an alkylcycloalkyl; a heteroalkylcycloalkyl; an aryl; a heteroaryl; an aralkyl; or a heteroaralkyl group;
L⁶ represents a C₁₋₆ alkyl, C₃₋₆ alkenyl, or C₃₋₆ alkynyl group, in which 1 to 5 H atoms may, independently of each other, be replaced by halogen atom, CN, CF₃, NO₂, OR or NHR, and/or in which one or two non-adjacent CH₂ group(s) may be replaced by O, NH, S, SO, SO₂, or C₃₋₇ cycloalkyl;
W represents a hydrogen atom; a C₁₋₆ alkyl, C₃₋₆ alkenyl, or C₃₋₆ alkynyl group, in which 1 to 5 H atoms may, independently of each other, be replaced by halogen atom, CN, CF₃, NO₂, OR or NHR, and/or in which one or two non-adjacent CH₂ group(s) may be replaced by O, NH, S, SO, SO₂, or C₃₋₇ cycloalkyl; NR⁵R⁶,-NHC(=O)R⁴, -NHSO₂R⁴, -C(O)R⁵, -(CH)[(CH₂)ₘC(O)OR⁴]₂; or a 5, 6, or 7-membered saturated, unsaturated or aromatic carbocyclic or heterocyclic ring, optionally substituted by 1, 2, or 3 R⁷;
R⁷, in each case, independently represents a halogen atom, an oxygen atom, a sulphur atom, CN, CF₃, NH₂, or NO₂;
X¹, X², and X³ each independently represents H or CH₃;
m each independently is 0, 1, 2, 3, or 4;
n each independently is an integer from 1 to 40; and
p each independently is 0, 1, 2 or 3.

2. The compound according to claim 1, or a pharmacologically acceptable salt thereof, wherein R¹, R² or R³ represents a group selected from the groups: 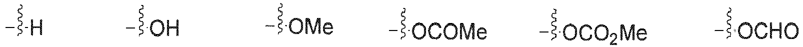 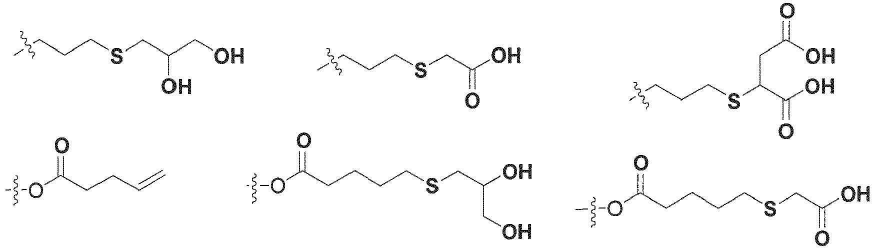 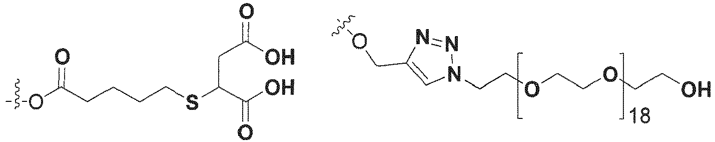 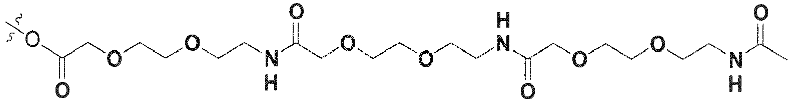 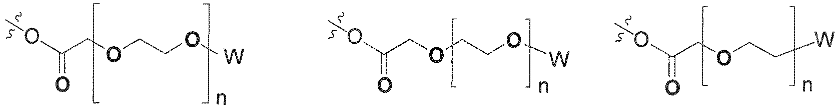 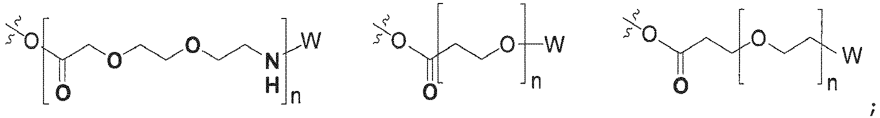 wherein n and W are defined as in claim 1.

3. The compound according to claim 1 or 2, or a pharmacologically acceptable salt thereof, wherein R¹ represents OH or OMe.

4. The compound according to any one of claims 1 to 3, or a pharmacologically acceptable salt thereof, wherein X¹ is CH₃ and X² is H.

5. The compound according to any one of claims 1 to 4, or a pharmacologically acceptable salt thereof, wherein A represents a group of the formula:

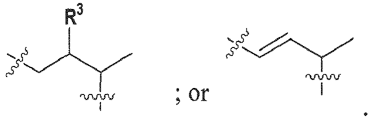

6. The compound according to claim 5, or a pharmacologically acceptable salt thereof, wherein R³ represents a hydrogen atom or a hydroxyl group.

7. The compound according to any one of claims 1 to 6, or a pharmacologically acceptable salt thereof, wherein the compound is represented by one of the following formulas: 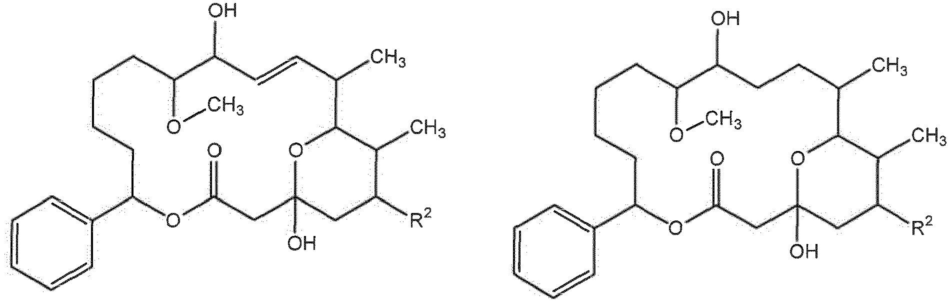 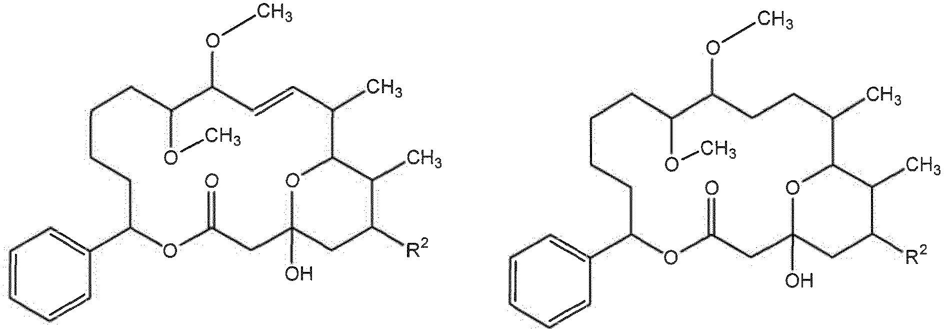 ; or 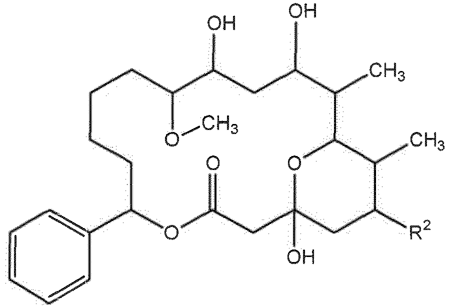 wherein R² is defined as in claim 1 or 2.

8. The compound according to any one of claims 1 to 7, or a pharmacologically acceptable salt thereof, wherein the compound is: 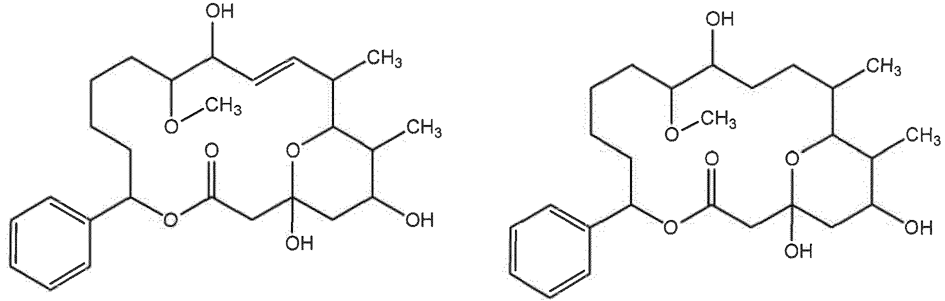 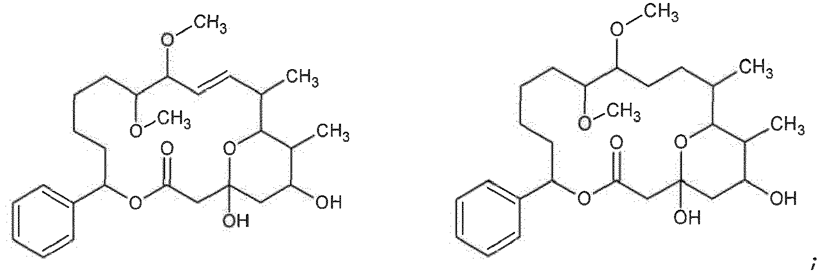 or

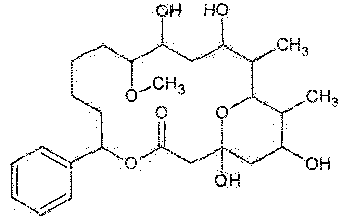

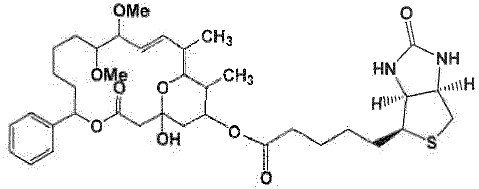

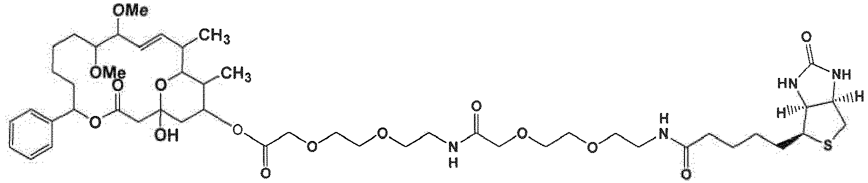

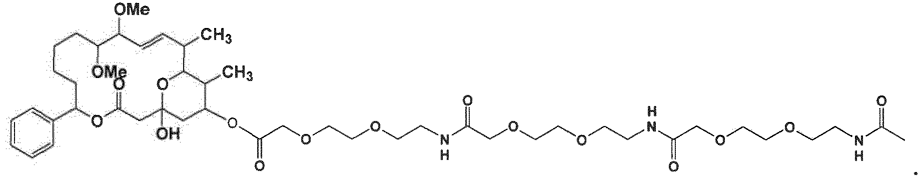

9. The compound according to any one of claims 1 to 4, or a pharmacologically acceptable salt thereof, wherein A represents a group of the formula:

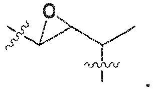

10. A pharmaceutical composition comprising at least one compound according to any one of claims 1 to 9 and, optionally, one or more carrier substance(s), excipient(s) and/or adjuvant(s).

11. A combination preparation containing at least one compound according to any one of claims 1 to 9, and at least one further active pharmaceutical ingredient.

12. The combination preparation of Claim 11, wherein the further active pharmaceutical ingredient is selected from (3*S*,6*R*,7*E*,9*R*,10*R*,12*R*,14*S*,15*E*,17*E*,19*E*,21*S*,23*S*,26*R*,27*R*,34a*S*)-9,10,12,13,14,21,22,23,24,25,26,27,32,33,34,34a-hexadecahydro-9,27-dihydroxy-3-[(1*R*)-2-[(1*S*,3*R*,4*R*)-4-hydroxy-3-methoxycyclohexyl]-1-methylethyl]-10,21-dimethoxy-6,8,12,14,20,26-hexamethyl-23,27-epoxy-3*H*-pyrido[2,1-c][1,4]-oxaazacyclohentriacontine-1,5,11,28,29(4*H*,6*H*,31*H*)-pentone (Rapamycin); AP-23481, (1*R*,2*R*,4*S*)-4-{(2*R*)-2-[(3*S*,6*R*,7*E*,9*R*,10*R*, 12*R*,14*S*,15*E*,17*E*,19*E*,21*S*,23*S*,26*R*,27*R*,34a*S*)-9,27-dihydroxy-10,21-dimethoxy-6,8,12,14,20,26-hexamethyl-1,5,11,28,29-pentaoxo-1,4,5,6,9,10,11,12,13,14,21,22,23,24,25,26,27,28,29, 31,32,33,34,34a-tetracosahydro-3H-23,27-epoxypyrido[2,1-*c*][1,4]oxazacyclohentriacontin-3-yl]propyl}-2-methoxycyclohexyl 3-hydroxy-2-(hydroxymethyl)-2-methylpropanoate (Temsirolimus), dihydroxy-12-[(2*R*)-1-[(1*S*,3*R*,4*R*)-4-(2-hydroxyethoxy)-3-methoxycyclohexyl]propan-2-yl]-19,30-dimethoxy-15,17,21,23,29,35-hexamethyl-11,36-dioxa-4-azatricyclo[30.3.1.0^{4,9}]hexatriaconta-16,24,26,28-tetraene-2,3,10,14,20-pentone (Everolimus); and (1*R*,2*R*,4*S*)-4-[(2*R*)-2-[(1*R,9S,*12*S,*15*R,*16*E*,18*R*,19*R*,21*R*,23*S*,24*E*,26*E*,28Z,30*S*,32*S*,35*R*)-1,18-dihydroxy-19,30-dimethoxy-15,17,21,23,29,35-hexamethyl-2,3,10,14,20-pentaoxo-11,36-dioxa-4-azatricyclo[30.3.1.0^{4,9}]hexa-triaconta-16,24,26,28-tetraen-12-yl]propyl]-2-methoxycyclohexyl dimethylphosphinate (Ridaforolimus); and antiviral drugs.

13. The compound according to any one of claims 1 to 9, the pharmaceutical composition according to claim 10, or the combination preparation according to claims 11 and 12 for use as a medicament.

14. The compound according to any one of claims 1 to 9, the pharmaceutical composition according to claim 10, or the combination preparation according to claims 11 and 12 for use in the treatment or prophylaxis of a Thl7-associated inflammatory and/or autoimmune disease.

15. The compound, the pharmaceutical composition, or the combination preparation for use according to claim 14, wherein the Th17-associated inflammatory and/or autoimmune disease is selected from: rheumatoid arthritis, psoriasis, systemic sclerosis, systemic lupus erythematosus, asthma, atopic dermatitis, contact hypersensitivity, multiple sclerosis, autoimmune myocarditis, type I diabetes, autoimmune thyroiditis, inflammatory bowel disease, Crohn's disease, ulcerative colitis, and transplant rejection.

16. The compound according to any one of claims 1 to 9, the pharmaceutical composition according to claim 10, or the combination preparation according to claims 11 and 12 for use in the treatment or prophylaxis of a viral infection.

17. A method for the preparation of a compound of formula (I), the method comprising the steps of:
(a) fermenting *Sorangium cellulosum* (DSM 28093); and
(b) separating and retaining the compound from the culture broth; wherein the compound is a compound according to claim 8.

## Patentansprüche

1. Verbindung der allgemeinen Formel (I): 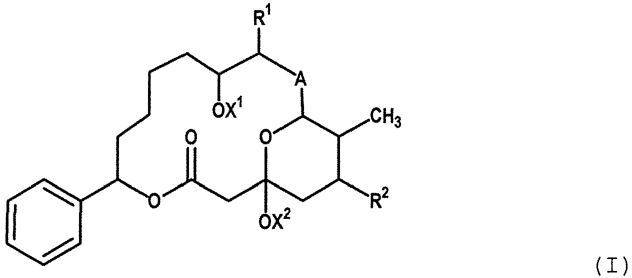 oder ein pharmazeutisch akzeptables Salz derselben, wobei
A eine Gruppe der Formel: 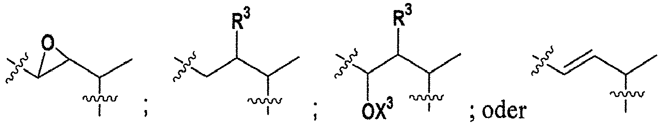 darstellt;
R¹, R² und R³, bei jedem Auftreten, jeweils unabhängig ein Wasserstoffatom; ein Halogenatom, CN, CF₃, NO₂, NH₂,-NHC(=O) R⁴, -NHSO₂R⁴, -(CH₂)ₘ-L¹-W, -O(CH₂)ₘ-L²-W, -C(O)(CH₂)ₘ-L³-W, -OC(O)(CH₂)ₘ-L³-W, OC(O)₂(CH₂)ₘ-L³-W, -(C₅₋₆ heterocyclyl)-L⁴-W, SOCH₃, SOCN, SOCF₃, SO₂CH₃, SO₂CN, SO₂CF₃, SO₂NR⁵R⁶, C(=O)NR⁵R⁶, COCH₃, COCF₃, C(CN)₃, 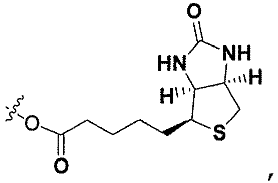 oder 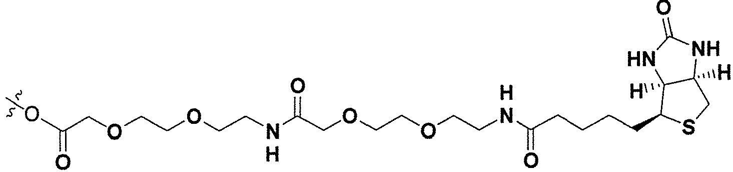 darstellt;
R⁴, bei jedem Auftreten, jeweils unabhängig ein Wasserstoffatom; eine Alkyl-; eine Alkenyl-; eine Alkynyl-; eine Heteroalkyl-; eine Cycloalkyl-; eine Heterocycloalkyl-; eine Aryl-;eine Heteroaryl-; eine Aralkyl-; oder eine Heteroaralkyl- Gruppe darstellt;
R⁵ und R⁶ jeweils unabhängig ein Wasserstoffatom; oder -(CH₂)ₘ-L⁵ darstellt; oder
R⁵ und R⁶ miteinander verbunden sind, um einen 5- bis 8-gliedrigen gesättigten, ungesättigten oder aromatischen Heterocyclus, welcher 1 bis 4 N- Atome oder 1 bis 3 Heteroatome, die aus der Gruppe, welche aus Stickstoff, Sauerstoff und Schwefel besteht, ausgewählt sind, enthält, zu bilden, wobei der Heterocyclus unsubstituiert oder mono-, di- oder trisubstituiert mit einem Halogenatom oder L⁵ sein kann; oder R⁵ und R⁶ miteinander verbunden sind, um einen 5- bis 8-gliedrigen gesättigten, ungesättigten oder aromatischen Heterocyclus, welcher 1 bis 3 Heteroatome, die aus der Gruppe, welche aus Stickstoff, Sauerstoff und Schwefel besteht, ausgewählt sind, enthält, und der an einen oder zwei Ringe, der / die aus der Gruppe, welche aus einem Cycloalkyl; einem Heterocycloalkyl; einem Alkylcycloalkyl; einem Heteroalkylcycloalkyl; einem Aryl; einem Heteroaryl; einem Aralkyl; und einem Heteroaralkyl besteht, ausgewählt ist / sind, kondensiert ist;
L¹, L², L³ und L⁴, bei jedem Auftreten, jeweils unabhängig eine Einzelbindung, O, S, NH, SO, SO₂, -O(CH₂CH₂O)ₙ-, -(OCH₂CH₂)ₙ-, oder -[C(O)CH₂-(OCH₂CH₂)₂-NH]ₙ- darstellt;
L⁵ ein H; -(CH₂)ₚ-L⁶; -(CH₂)ₚ-OL⁶; eine C₁₋₆ Heteroalkyl-; eine Cycloalkyl-; eine Heterocycloalkyl-; eine Alkylcycloalkyl-; eine Heteroalkylcycloalkyl-; eine Aryl-; eine Heteroaryl-; eine Aralkyl-; oder eine Heteroaralkyl- Gruppe darstellt;
L⁶ eine C₁₋₆ Alkyl-, eine C₃₋₆ Alkenyl-, oder eine C₃₋₆ Alkynyl-Gruppe, in denen 1 bis 5 H- Atome, jeweils unabhängig voneinander, durch ein Halogenatom, CN, CF₃, NO₂, OR oder NHR ersetzt sein kann / können, und / oder in denen eine CH₂-Gruppe oder zwei nicht benachbarte CH₂- Gruppen durch O, NH, S, SO, SO₂ oder C₃₋₇- Cycloalkyl ersetzt sein kann / können, darstellt;
W ein Wasserstoffatom; eine C₁₋₆ Alkyl-, eine C₃₋₆ Alkenyl-, oder eine C₃₋₆ Alkynyl- Gruppe, in denen 1 bis 5 H- Atome, jeweils unabhängig voneinander, durch ein Halogenatom, CN, CF₃, NO₂, OR oder NHR ersetzt sein kann / können, und / oder in denen eine CH₂- Gruppe oder zwei nicht benachbarte CH₂- Gruppen durch O, NH, S, SO, SO₂ oder C₃₋₇- Cycloalkyl ersetzt sein kann / können;; -NR⁵R⁶, -NHC(=O)R⁴, -NHSO₂R⁴, -C(O)R⁵,-(CH)[(CH₂)ₘC(O)OR⁴]₂; oder einen 5, 6, oder 7-gliedrigen gesättigten, ungesättigten oder aromatischen carbocyclischen oder heterocyclischen Ring, der gegebenenfalls mit 1, 2 oder 3 R⁷ substituiert ist, darstellt;
R⁷, bei jedem Auftreten, jeweils unabhängig ein Halogenatom, ein Sauerstoffatom, ein Schwefelatom, CN, CF₃, NH₂, oder NO₂ darstellt;
X¹, X², und X³ jeweils unabhängig voneinander ein H oder CH₃ darstellt;
m jeweils unabhängig 0, 1, 2, 3, oder 4 ist;
n jeweils unabhängig eine ganze Zahl von 1 bis 40 ist; und
p jeweils unabhängig 0, 1, 2 oder 3 ist.

2. Die Verbindung nach Anspruch 1, oder ein pharmazeutisch akzeptables Salz derselben, wobei R¹, R² oder R³ eine Gruppe darstellt, die ausgewählt ist aus den Gruppen: 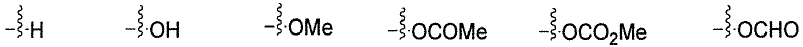 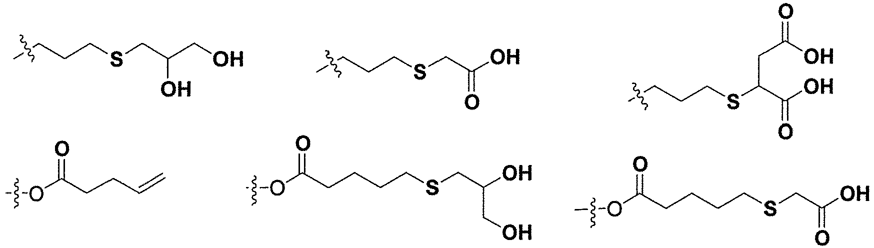 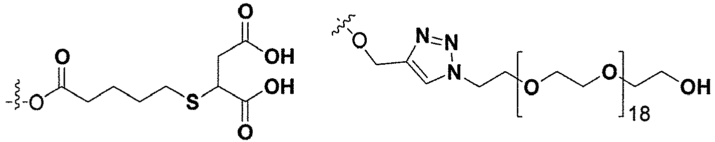 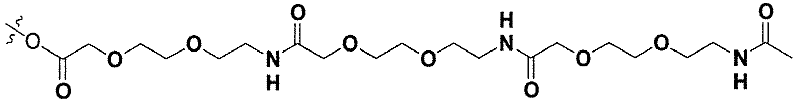 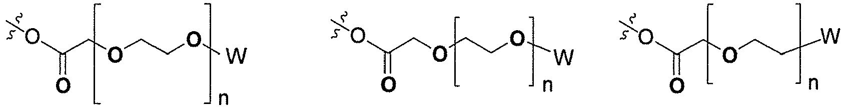 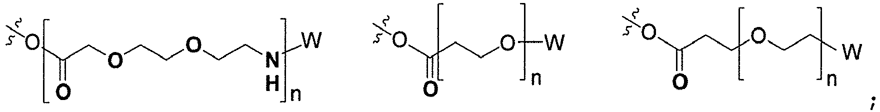 wobei n und W wie in Anspruch 1 definiert sind.

3. Die Verbindung nach Anspruch 1 oder 2, oder ein pharmazeutisch akzeptables Salz derselben, wobei R¹ OH oder OMe darstellt.

4. Die Verbindung nach einem der Ansprüche 1 bis 3, oder ein pharmazeutisch akzeptables Salz derselben, wobei X¹ CH₃ ist und X² H ist.

5. Die Verbindung nach einem der Ansprüche 1 bis 4, oder ein pharmazeutisch akzeptables Salz derselben, wobei A eine Gruppe der Formel: 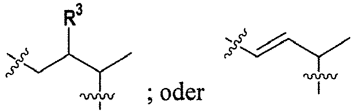 darstellt.

6. Die Verbindung nach Anspruch 5, oder ein pharmazeutisch akzeptables Salz derselben, wobei R³ ein Wasserstoffatom oder eine Hydroxylgruppe darstellt.

7. Die Verbindung nach einem der Ansprüche 1 bis 6, oder ein pharmazeutisch akzeptables Salz derselben, wobei die Verbindung durch eine der folgenden Formeln dargestellt ist: 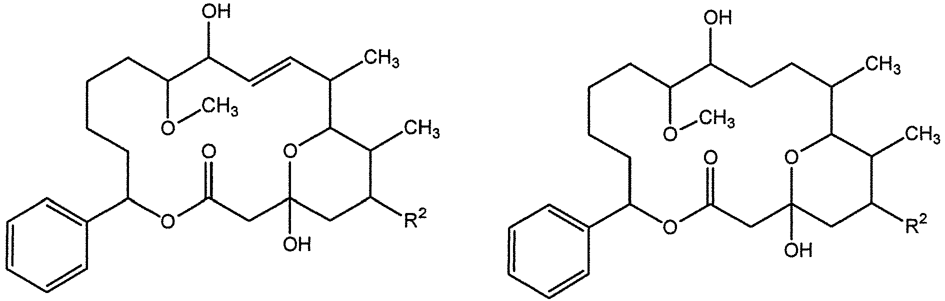 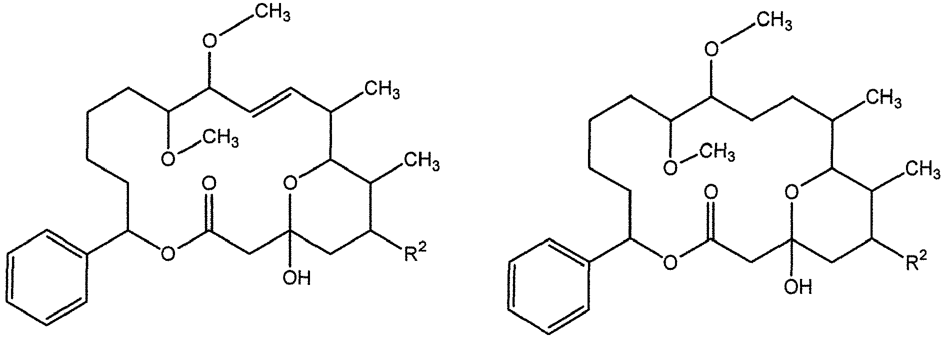 ; oder 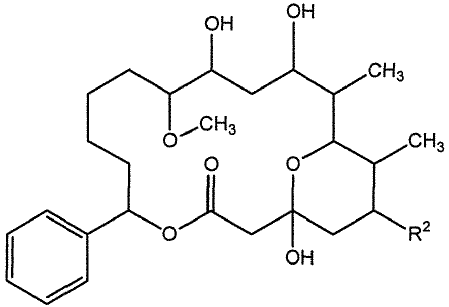 wobei R² wie in Anspruch 1 oder 2 definiert ist.

8. Die Verbindung nach einem der Ansprüche 1 bis 7, oder ein pharmazeutisch akzeptables Salz derselben, wobei die Verbindung: 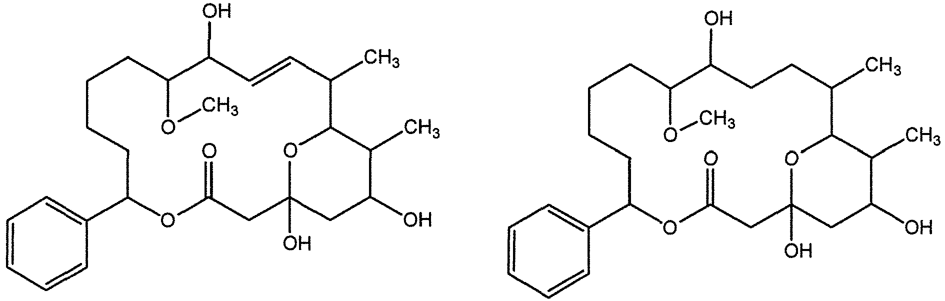 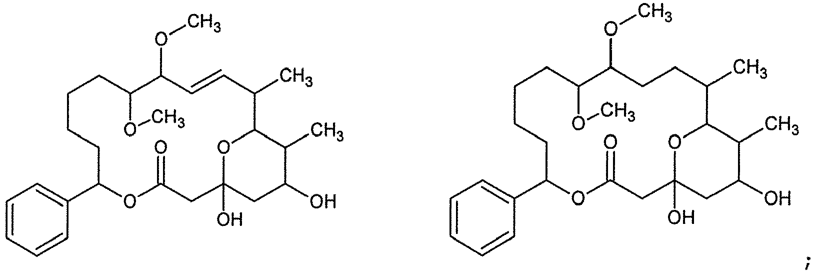 oder 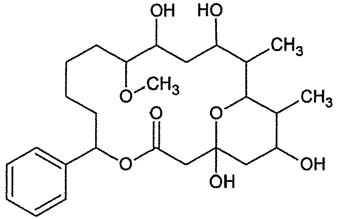 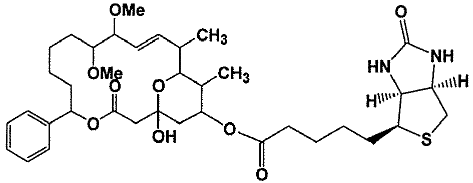 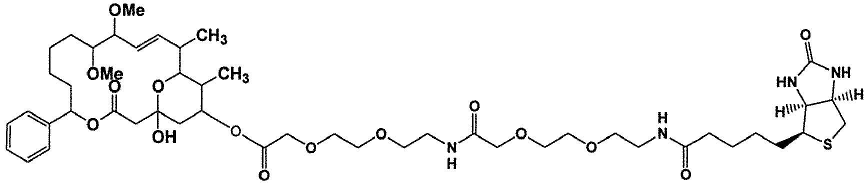 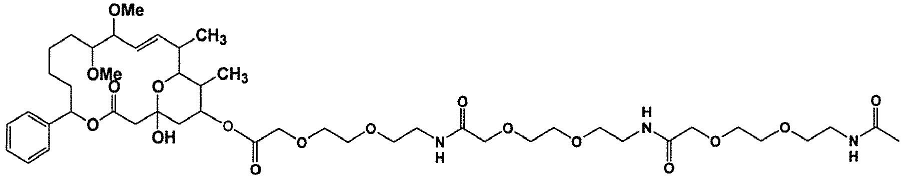 ist.

9. Die Verbindung nach einem der Ansprüche 1 bis 4, oder ein pharmazeutisch akzeptables Salz derselben, wobei A eine Gruppe der Formel: 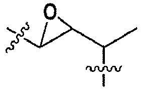 darstellt.

10. Pharmazeutische Zusammensetzung, die mindestens eine Verbindung gemäß einem der Ansprüche 1 bis 9 und gegebenenfalls eine oder mehrere Trägersubstanz(en), (einen) Arzneistoffträger und / oder (einen) Hilfsstoff(e) umfasst.

11. Kombinationspräparat, das mindestens eine Verbindung gemäß einem der Ansprüche 1 bis 9 und mindestens einen weiteren pharmazeutisch aktiven Wirkstoff enthält.

12. Das Kombinationspräparat nach Anspruch 11, wobei der weitere pharmazeutisch aktive Wirkstoff aus (3*S*,6*R*,7*E*,9*R*,10*R*,12*R*,14*S*,15*E*,17*E*,19*E*,21*S*,23*S*,26*R*,27*R,*34a*S*)-9,10,12,13,14,21,22,23,24,25,26,27,32,33,34,34a-Hexadecahydro-9,27-dihydroxy-3-[(1*R*)-2-[(1*S*,3*R*,4*R*)-4-hydroxy-3-methoxycyclohexyl]-1-methylethyl]-10,21-dimethoxy-6,8,12,14,20,26-hexamethyl-23,27-epoxy-3*H*-pyrido[2,1-c][1,4]-oxaazacyclohentriacontin-1,5,11,28,29(4*H*,6*H*,31*H*)-penton (Rapamycin); AP-23481, (1*R*,2*R*,4*S*)-4-{(2*R*)-2-[(3*S*,6*R*,7*E*,9*R*,10*R*, 12*R*,14*S*,15*E*,17*E*,19*E*,21*S*,23*S*,26*R*,27*R*,34a*S*)-9,27-Dihydroxy-10,21-dimethoxy-6,8,12,14,20,26-hexamethyl-1,5,11,28,29-pentaoxo-1,4,5,6,9,10,11,12,13,14,21,22,23,24,25,26,27,28,29, 31,32,33,34,34a-tetracosahydro-3*H*-23,27-epoxypyrido[2,1-*c*][1,4]oxazacyclohentriacontin-3-yl]propyl}-2-methoxycyclohexyl 3-hydroxy-2-(hydroxymethyl)-2-methylpropanoat (Temsirolimus), Dihydroxy-12-[(2*R*)-1-[(1*S*,3*R*,4*R*)-4-(2-hydroxyethoxy)-3-methoxycyclohexyl]propan-2-yl]-19,30-dimethoxy-15,17,21,23,29,35-hexamethyl-11,36-dioxa-4-azatricyclo[30.3.1.0^{4,9}]hexatriaconta-16,24,26,28-tetraen-2,3,10,14,20-penton (Everolimus); und (1*R*,2*R*,4*S*)-4-[(2*R*)-2-[(1*R*,9*S*,12*S*,15*R*,16*E*,18*R*,19*R*,21*R*,23*S,*24*E*,26*E*,28*Z*,30*S*,32*S*,35*R*)-1,18-Dihydroxy-19,30-dimethoxy-15,17,21,23,29,35-hexamethyl-2,3,10,14,20-pentaoxo-11,36-dioxa-4-azatricyclo[30.3.1.0⁴,⁹]hexa-triaconta-16,24,26,28-tetraen-12-yl]propyl]-2-methoxycyclohexyl dimethylphosphinat (Ridaforolimus); und antiviralen Arzneimitteln ausgewählt ist.

13. Die Verbindung gemäß einem der Ansprüche 1 bis 9, die pharmazeutische Zusammensetzung gemäß Anspruch 10 oder das Kombinationspräparat gemäß den Ansprüchen 11 und 12 zur Verwendung als Medikament.

14. Die Verbindung gemäß einem der Ansprüche 1 bis 9, die pharmazeutische Zusammensetzung gemäß Anspruch 10 oder das Kombinationspräparat gemäß den Ansprüchen 11 und 12 zur Verwendung bei der Behandlung oder Prophylaxe einer mit Th17 in Verbindung stehenden Entzündungs- und / oder Autoimmunerkrankung.

15. Die Verbindung, die pharmazeutische Zusammensetzung, das Kombinationspräparat zur Verwendung gemäß Anspruch 14, wobei die mit Th17 in Verbindung stehende Entzündungs- und / oder Autoimmunerkrankung aus: rheumatoider Arthritis, Psoriasis, systemischer Sklerose, systemischem Lupus erythematodes, Asthma, atopischer Dermatitis, Kontaktüberempfindlichkeit, Multipler Sklerose, Autoimmun- Myokarditis, Diabetes Typ I, Autoimmun- Thyreoiditis, einer entzündlichen Darmerkrankung, Morbus Crohn, Colitis ulcerosa und einer Transplantatabstoßung ausgewählt ist.

16. Die Verbindung gemäß einem der Ansprüche 1 bis 9, die pharmazeutische Zusammensetzung gemäß Anspruch 10 oder das Kombinationspräparat gemäß den Ansprüchen 11 und 12 zur Verwendung bei der Behandlung oder Prophylaxe einer Virusinfektion.

17. Verfahren zur Herstellung einer Verbindung der Formel (I), wobei das Verfahren die Schritte umfasst:
(a) das Fermentieren von *Sorangium cellulosum* (DSM 28093); und
(b) das Abtrennen und Gewinnen der Verbindung aus dem Kulturmedium; wobei die Verbindung eine Verbindung gemäß Anspruch 8 ist.

## Revendications

1. Composé de formule générale (I) : 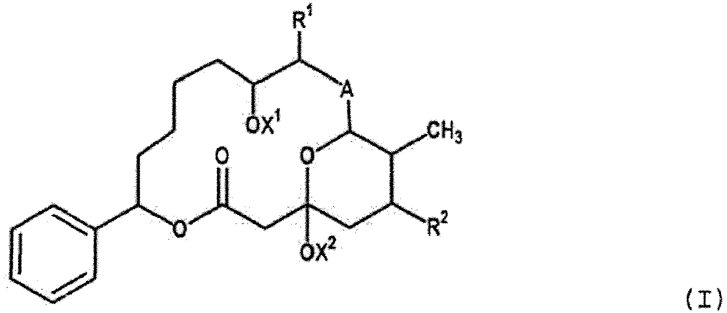 ou l'un de ses sels pharmacologiquement acceptables, dans lequel A représente un groupe de formule : 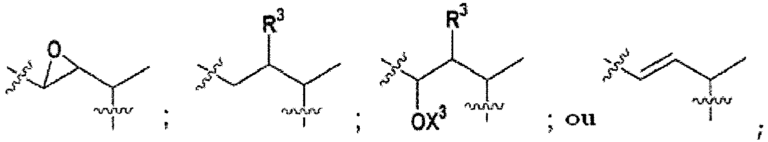 R¹, R² et R³, dans chaque cas, représentent indépendamment un atome d'hydrogène, un atome d'halogène, un groupe CN, CF₃, NO₂, NH₂, -NHC(=O)R⁴, -NHSO₂R⁴, -(CH₂)ₘ-L¹-W, -O(CH₂)ₘ-L²-W, -C(O)(CH₂)ₘ-L³-W, -OC(O)(CH₂)ₘ-L³-W, OC(O)₂(CH₂)ₘ-L³-W, - (hétérocyclyl en C₅ à C₆)-L⁴-W, SOCH₃, SOCN, SOCF₃, SO₂CH₃, SO₂CN, SO₂CF₃, SO₂NR⁵R⁶, C(=O)NR⁵R⁶, COCH₃, COCF₃, ou C(CN)₃, 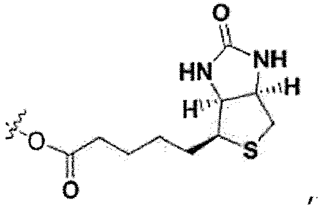 ou 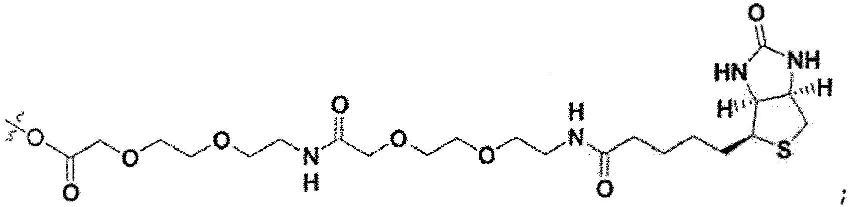
R⁴, dans chaque cas, représente indépendamment un atome d'hydrogène ; un groupe alkyle ; alcényle ; alcynyle ; hétéroalkyle ; cycloalkyle ; hétérocycloalkyle ; aryle ; hétéroaryle ; aralkyle ; ou hétéroaralkyle ;
R⁵ et R⁶ représentent chacun indépendamment un atome d'hydrogène ; ou -(CH₂)ₘ-L⁵ ; ou
R⁵ et R⁶ sont pris ensemble pour former un hétérocycle saturé, insaturé ou aromatique de 5 à 8 chaînons contenant 1 à 4 atomes N ou 1 à 3 hétéroatomes choisis dans le groupe constitué d'azote, d'oxygène et de soufre, lequel hétérocycle peut être non substitué ou mono-, di- ou tri-substitué par un atome d'halogène ou L⁵ ; ou R⁵ et R⁶ sont pris ensemble pour former un hétérocycle saturé, insaturé ou aromatique de 5 à 8 chaînons contenant 1 à 3 hétéroatomes choisis dans le groupe constitué d'azote, d'oxygène et de soufre, qui est fusionné à un ou deux cycles choisis dans le groupe constitué de cycles cycloalkyle ; hétérocycloalkyle ; alkylcycloalkyle ; hétéroalkylcycloalkyle ; aryle ; hétéroaryle ; aralkyle ; et hétéroaralkyle ;
L¹, L², L³ et L⁴, dans chaque cas, représentent indépendamment une liaison simple, O, S, NH, SO, SO₂, -O(CH₂CH₂O)ₙ-, -(OCH₂CH₂)ₙ-, ou -[C(O)CH₂-(OCH₂CH₂)₂-NH]ₙ- ;
L⁵ représente H; -(CH₂)ₚ-L⁶ ; -(CH₂)ₚ-OL⁶ ; un groupe hétéroalkyle en C₁ à C₆ ; cycloalkyle ; hétérocycloalkyle ; alkylcycloalkyle ; hétéroalkylcycloalkyle ; aryle ; hétéroaryle ; aralkyle ; ou hétéroaralkyle ;
L⁶ représente un groupe alkyle en C₁ à C₆, alcényle en C₃ à C₆, ou alcynyle en C₃ à C₆, dans lequel 1 à 5 atomes H, indépendamment les uns des autres, peuvent être remplacés par un atome d'halogène, un groupe CN, CF₃, NO₂, OR ou NHR, et/ou dans lequel un ou deux groupes CH₂ non adjacents peuvent être remplacés par O, NH, S, SO, SO₂, ou un groupe cycloalkyle en C₃ à C₇ ;
W représente un atome d'hydrogène ; un groupe alkyle en C₁ à C₆, alcényle en C₃ à C₆, ou alcynyle en C₃ à C₆, dans lequel 1 à 5 atomes H, indépendamment les uns des autres, peuvent être remplacés par un atome d'halogène, un groupe CN, CF₃, NO₂, OR ou NHR, et/ou dans lequel un ou deux groupes CH₂ non adjacents peuvent être remplacés par O, NH, S, SO, SO₂, ou un groupe cycloalkyle en C₃ à C₇ ; NR⁵R⁶, -NHC(=O)R⁴, -NHSO₂R⁴, -C(O)R⁵,-(CH)[(CH₂)ₘC(O)OR⁴]₂ ; ou un cycle carbocyclique ou hétérocyclique saturé, insaturé ou aromatique de 5, 6 ou 7 chaînons, éventuellement substitué par 1, 2, ou 3 R⁷ ;
R⁷, dans chaque cas, représente indépendamment un atome d'halogène, un atome d'oxygène, un atome de soufre, CN, CF₃, NH₂, ou NO₂ ;
X¹, X², et X³ représentent chacun indépendamment H ou CH₃ ;
m chacun indépendamment vaut 0, 1, 2, 3, ou 4 ;
n chacun indépendamment est un nombre entier de 1 à 40 ; et
p chacun indépendamment vaut 0, 1, 2 ou 3.

2. Composé selon la revendication 1, ou l'un de ses sels pharmacologiquement acceptables, dans lequel R¹, R² ou R³ représente un groupe choisi parmi les groupes : 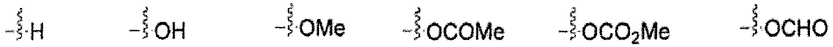 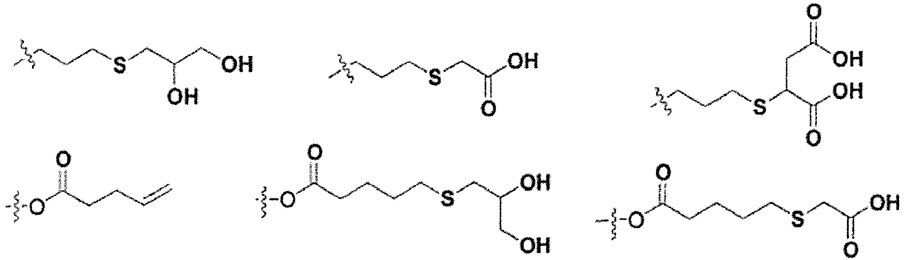 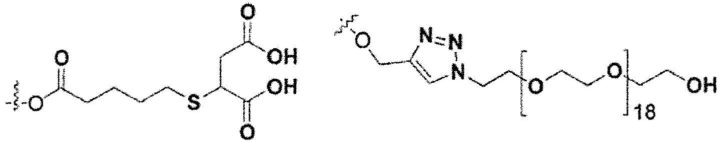 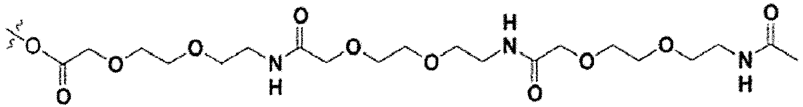 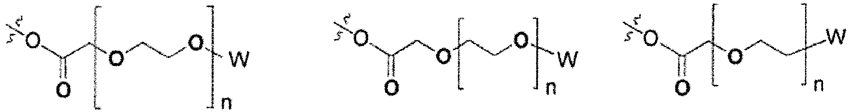 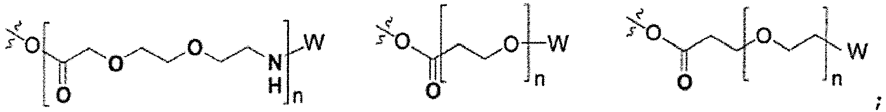 dans lequel n et W sont définis comme dans la revendication 1.

3. Composé selon la revendication 1 ou 2, ou l'un de ses sels pharmacologiquement acceptables, dans lequel R¹ représente OH ou OMe.

4. Composé selon l'une quelconque des revendications 1 à 3, ou l'un de ses sels pharmacologiquement acceptables, dans lequel X¹ représente CH₃ et X² représente H.

5. Composé selon l'une quelconque des revendications 1 à 4, ou l'un de ses sels pharmacologiquement acceptables, dans lequel A représente un groupe de formule :

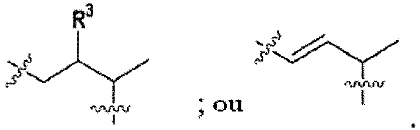

6. Composé selon la revendication 5, ou l'un de ses sels pharmacologiquement acceptables, dans lequel R³ représente un atome d'hydrogène ou un groupe hydroxyle.

7. Composé selon l'une quelconque des revendications 1 à 6, ou l'un de ses sels pharmacologiquement acceptables, dans lequel le composé est représenté par l'une des formules suivantes : 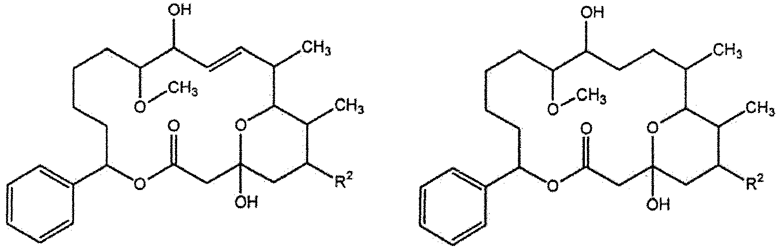 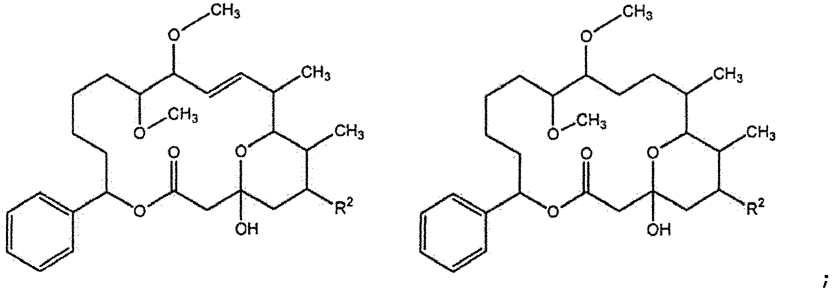 ou 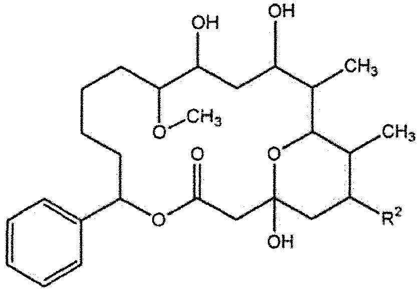 dans lequel R² est défini comme dans la revendication 1 ou 2.

8. Composé selon l'une quelconque des revendications 1 à 7, ou l'un de ses sels pharmacologiquement acceptables, dans lequel le composé est : 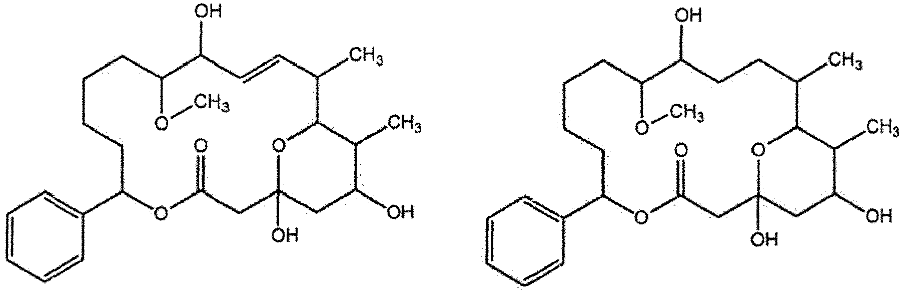 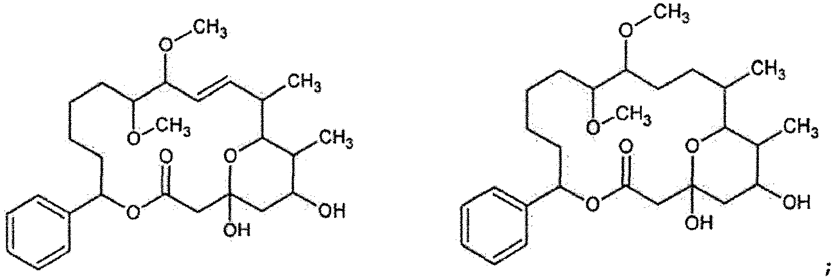 ou

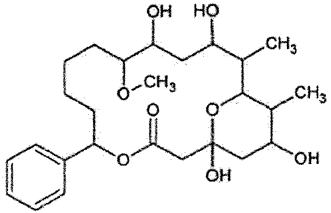

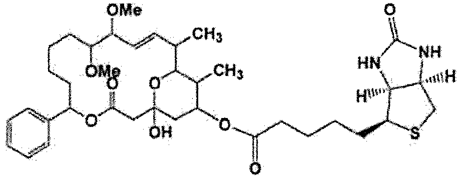

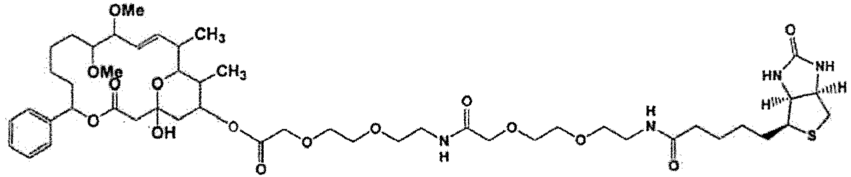

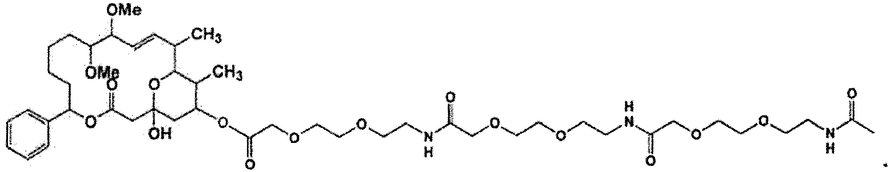

9. Composé selon l'une quelconque des revendications 1 à 4, ou l'un de ses sels pharmacologiquement acceptables, dans lequel A représente un groupe de formule :

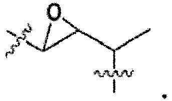

10. Composition pharmaceutique comprenant au moins un composé selon l'une quelconque des revendications 1 à 9 et, éventuellement, une ou plusieurs substances de support, un ou plusieurs excipients et/ou adjuvants.

11. Préparation combinée contenant au moins un composé selon l'une quelconque des revendications 1 à 9, et au moins un autre principe actif pharmaceutique.

12. Préparation combinée selon la revendication 11, dans laquelle l'autre principe actif pharmaceutique est choisi parmi la (3*S*,6*R*,7*E*,9*R*,10*R*,12*R*,14*S*,15*E*,17*E*,19*E*,21*S*,23*S*,26*R*,27*R,*34a*S*)-9,10,12,13,14,21,22,23,24,25,26,27,32,33,34,34a-hexadécahydro-9,27-dihydroxy-3-[(1*R*)-2-[(1*S*,3*R*,4*R*)-4-hydroxy-3-méthoxycyclohexyl]-1-méthyléthyl]-10,21-diméthoxy-6,8,12,14, 20,26-hexaméthyl-23,27-époxy-3*H*-pyrido[2,1-c][1,4]-oxaaza-cyclohentriacontine-1,5,11,28,29(4*H*,6*H*,31*H*)-pentone (Rapamycine) ; AP-23481, le 3-hydroxy-2-(hydroxyméthyl)-2-méthyl-propanoate de (1*R*,2*R*,4*S*)-4-{(2*R*)-2-[(3*S*,6*R*,7*E*,9*R*,10*R*, 12*R*,14*S*,15*E*,17*E*,19*E*,21*S*,23*S*,26*R*,27*R*,34a*S*)-9,27-dihydroxy-10,21-diméthoxy-6,8,12,14,20,26-hexaméthyl-1,5,11,28,29-pentaoxo-1,4,5,6,9,10,11,12,13,14,21,22,23,24,25,26,27,28,29, 31,32,33,34,34a-tétracosahydro-3*H*-23,27-époxypyrido[2,1-c]-[1,4]oxaza-cyclohentriacontin-3-yl]propyl}-2-méthoxy-cyclohexyle (Temsirolimus), la dihydroxy-12-[(2*R*)-1-[(1*S*,3*R*,4*R*)-4-(2-hydroxyéthoxy)-3-méthoxycyclohexyl]propan-2-yl]-19,30-diméthoxy-15,17,21,23,29,35-hexaméthyl-11,36-dioxa-4-azatricyclo[30.3.1.0^{4,9}]hexatriaconta-16,24,26,28-tétraène-2,3,10,14,20-pentone (Evérolimus) ; et le diméthylphosphinate de (1*R*,2*R*,4*S*)-4-[(2*R*)-2-[(1*R*,9*S*,12*S*,15*R*,16*E*,18*R*,19*R*,21*R*,23*S*, 24*E*,26*E*,28*Z*,30*S*,32*S*,35*R*)-1,18-dihydroxy-19,30-diméthoxy-15,17,21,23,29,35-hexaméthyl-2,3,10,14,20-pentaoxo-11,36-dioxa-4-azatricyclo[30.3.1.0^{4,9}]hexatriaconta-16,24,26,28-tetraén-12-yl]-propyl]-2-méthoxycyclohexyle (Ridaforolimus) ; et des médicaments antiviraux.

13. Composé selon l'une quelconque des revendications 1 à 9, composition pharmaceutique selon la revendication 10, ou préparation combinée selon les revendications 11 et 12 destiné à une utilisation en tant que médicament.

14. Composé selon l'une quelconque des revendications 1 à 9, composition pharmaceutique selon la revendication 10, ou préparation combinée selon les revendications 11 et 12 destiné à une utilisation dans le traitement ou la prophylaxie d'une maladie inflammatoire et/ou auto-immune associée au Th17.

15. Composé, composition pharmaceutique, ou préparation combinée destiné à une utilisation selon la revendication 14, dans lequel la maladie inflammatoire et/ou auto-immune associée au Th17 est choisie parmi : la polyarthrite rhumatoïde, le psoriasis, la sclérose systémique, le lupus érythémateux systémique, l'asthme, la dermatite atopique, l'hypersensibilité de contact, la sclérose en plaques, la myocardite auto-immune, le diabète de type I, la thyroïdite auto-immune, une maladie inflammatoire de l'intestin, la maladie de Crohn, la rectocolite hémorragique, et le rejet de transplant.

16. Composé selon l'une quelconque des revendications 1 à 9, composition pharmaceutique selon la revendication 10, ou préparation combinée selon les revendications 11 et 12 destiné à une utilisation dans le traitement ou la prophylaxie d'une infection virale.

17. Procédé pour la préparation d'un composé de formule (I), le procédé comprenant les étapes de :
(a) fermentation de *Sorangium cellulosum* (DSM 28093) ; et
(b) séparation et rétention du composé à partir du bouillon de culture ; dans lequel le composé est un composé selon la revendication 8.
